(19)

**Europäisches Patentamt**
**European Patent Office**
**Office européen des brevets**

(11) **EP 4 122 903 A1**

(12) **EUROPEAN PATENT APPLICATION**

(43) Date of publication:
**25.01.2023 Bulletin 2023/04**

(21) Application number: **21186780.9**

(22) Date of filing: **20.07.2021**

(51) International Patent Classification (IPC):
$C04B\ 35/515^{(2006.01)}$   $A61L\ 27/04^{(2006.01)}$
$A61L\ 27/10^{(2006.01)}$   $A61L\ 27/14^{(2006.01)}$
$A61L\ 27/40^{(2006.01)}$   $A61L\ 27/48^{(2006.01)}$
$A61L\ 27/56^{(2006.01)}$   $B22C\ 9/02^{(2006.01)}$
$B22C\ 9/04^{(2006.01)}$   $B22D\ 19/14^{(2006.01)}$
$B22D\ 31/00^{(2006.01)}$   $C04B\ 35/626^{(2006.01)}$
$C04B\ 35/628^{(2006.01)}$   $C04B\ 35/638^{(2006.01)}$
$C04B\ 38/04^{(2006.01)}$   $C04B\ 35/632^{(2006.01)}$

(52) Cooperative Patent Classification (CPC):
(C-Sets available)
C04B 35/6261; A61L 27/10; A61L 27/427;
A61L 27/48; A61L 27/56; B22C 1/10; B22C 9/02;
B22C 9/04; B22C 9/105; B22D 18/06;
B22D 29/002; B33Y 80/00; C04B 35/5152;
C04B 35/6264; C04B 35/6269;          (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **ETH Zurich**
  **8092 Zurich (CH)**
• **Technische Universiteit Delft**
  **2628 CN Delft (NL)**

(72) Inventors:
• **STUDART, André R.**
  **8044 Zürich (CH)**
• **MASANIA, Kunal**
  **2342 AX Oegstgeest (NL)**
• **KLEGER, Nicole**
  **8104 Weiningen (CH)**
• **FEHLMANN, Simona**
  **8046 Zürich (CH)**
• **CIHOVA, Martina**
  **London, SW6 2RX (GB)**

(54) **METHOD FOR THE ADDITIVE MANUFACTURING OF CASTING MOLDS**

(57)     A method for manufacturing a photopolymerizable slurry comprises the steps of: providing a plurality of particles of an inorganic salt in the form of a powder; wherein at atmospheric pressure the inorganic salt has a melting point of above 250 °C; and wherein at room temperature the inorganic salt has a solubility in water above 9 %w/w; providing at least one radiation curable monomer; the at least one radiation curable monomer being in the liquid phase; and adding the inorganic salt particles to the liquid composition and mixing the inorganic salt particles with the liquid composition; obtaining a photopolymerizable slurry, selectively curing the photopolymerizable slurry to obtain a green body article; debinding the green body article to obtain a binderless body article; and sintering the binderless body article to obtain a sintered ceramic article. Providing a first template mold (1); wherein the first template mold comprises the sintered ceramic article. Providing a second mold (2, 2', 2"); wherein the second mold comprises a compartment into which said first template mold can be placed; mounting the first template mold into the compartment of the second mold, thereby obtaining an operative mold (1, 2, 2', 2"); casting a fluid casting material (3, 3', 3") into said operative mold to obtain after solidification of said casting material an infiltrated template mold (8) comprising a solid article (9) that is at least partially located within the first template mold (1); and separating said solid article from the first template mold by dissolving the sintered ceramic article of the first template mold with a suitable solvent, for example water.

**(Cont. next page)**

**Fig. 4**

(52) Cooperative Patent Classification (CPC): (Cont.)
**C04B 35/62802; C04B 35/632; C04B 35/638;
C04B 38/045;** C04B 2235/3201;
C04B 2235/6026; C04B 2235/6028;
C04B 2235/616

C-Sets
**C04B 38/045, C04B 35/00**

## Description

### Field of the invention

The invention relates to photopolymerizable slurries, methods for manufacturing such photopolymerizable slurries, methods for manufacturing sintered ceramic articles with such photopolymerizable slurries, and a method for casting articles.

### Background of the invention

The manufacturing of structures with controlled chemical composition, complex shape and porosity is crucial for many applications in medicine, engineering, and architecture. Because shaping technologies are not always available for the materials/chemical compositions of interest, it is sometimes convenient to manufacture templating molds into which materials with desired chemistry can be cast. Additive manufacturing has been extensively employed to fabricate such complex-shaped molds. However, removal of the mold after casting might be challenging or impossible when the final articles feature complex shapes or internal porosity.

Porosity is an essential feature in a wide range of applications that combine light weight with high surface area and tunable density. Porosity is desired in a broad range of materials for applications such as catalytic supports and lightweight structures. Particularly, porous materials are of high interest in the biomedical field, especially for their use in tissue engineering and bioresorbable implants, such as implants for osteosynthesis.

Highly porous materials can be obtained by several approaches such as salt leaching, gas foaming, freeze-drying and sintering, and phase separation [1].

With the salt leaching technique, porous materials based on all material classes, i.e. metals, ceramics or polymers, can be obtained [2]. Typically, salt particles are used as leachable powder to produce a compacted preform, which is then infiltrated with the material of interest. Upon solidification of the infiltrate, the salt is removed by leaching in a suitable solvent, and the solidified, infiltrated material remains. The salt leaching technique has been used to achieve porosity in a wide variety of materials, including natural polymers such as silk fibroin [3], synthetic polymers such as poly(l-lactic acid) [4], and bulk metallic glasses [5], and crystalline metals such as aluminum [6] and magnesium [7]. In all these approaches, the pore size of the final part is defined by the size of the original salt particles, or the salt particle aggregates used as the template. The pore geometry of the resulting material is random, with broad pore-size distributions, which reflects the polydisperse nature of the salt particle template matrix. This limits this technique's ability to control the porous architecture and achievable overall porosity of the final scaffold.

Additive manufacturing allows to manufacture complex three-dimensional shapes and is also used in the manufacturing of porous materials, where it allows tuning of the pore geometries and sizes. Additive manufacturing techniques allow to produce three-dimensional structures with well-controlled porosity and pore sizes from nanoscale to macroscale [8, 9, 10, 11, 12]. Various additive manufacturing techniques such as selective laser sintering (SLS), fused deposition modeling (FDM), selective laser melting (SLM) or stereolithography (SL) have been applied. SLS and FDM have been mainly applied for polymeric materials and as a combination with ceramics, e.g. polycaprolactone (PCL), polylactic acid (PLA), hydroxyapatite (HA), and calcium phosphate (CaP). SLM has been used for the fabrication of metal parts from metal powder, e.g. Mg. Moreover, porous parts from PCL and CaP were also achieved with stereolithography printing. [13, 14, 15]

While there is a broad choice of materials available for additive manufacturing, it is difficult to find materials that are both suitable for common AM techniques and result in a product having a mechanical strength suitable for the end application (e.g. as bone replacement) and still being biodegradable. It also remains a challenge to shape materials that possess a high chemical reactivity, such as for example magnesium, using additive manufacturing techniques.

Magnesium (Mg) is receiving increasing attention as a metallic biodegradable implant material for temporary bone replacement or osteosynthesis [16]. Magnesium has similar mechanical properties as bone and is able to induce new bone formation [17] and is bioresorbable [18]. It is widely accepted that pore size, pore shape, pore directionality, and the degree of porosity of Mg scaffolds are factors that strongly influence cell viability and growth. To guide bone-tissue growth, large open porosity with pore sizes >300 $\mu$m in combination with surface roughness appears to be most successful. [19, 20] Thus, the ability to shape the magnesium into structures with controlled porosity and pore size, and in a patient-specific outer geometry, is highly desired.

A three-step process has been reported for indirect additive manufacturing of metallic magnesium [21, 22]. A 3D polymer structure is produced via AM. The resulting polymer structure is then infiltrated with a NaCl paste. Upon removal of the polymer structure, the NaCl acts as a template for Mg infiltration. In this technique, the additional processing step required to generate the polymer template increases the processing time and results in enhanced risk of imperfect structure replication. Furthermore, the technique is limited to geometries that allow the necessary infiltration of the highly viscous NaCl paste into the polymer template.

WO 2020/046687 A1 discloses a method of making a non-oxide ceramic part. A photopolymerizable slurry, containing non-oxide ceramic particles with high melting point such a silicon carbide or boron nitride, is selectively cured to obtain a gelled article. The gelled article is dried to form an aerogel article or a xerogel article. The aerogel article or xerogel article is heat treated to form a porous ceramic article, which is then sintered to obtain a sintered ceramic article.

N. Kleger et al. [23] describe a method for directly 3D printing a NaCl template for Mg infiltration, using an ink consisting of NaCl particles dispersed in paraffin oil. In this method, a three-dimensional structure with controlled pore geometry can be printed via the direct ink writing technique. After removal of the paraffin oil, the resulting green body is calcined and sintered to a NaCl template. The template is infiltrated with melted magnesium. After leaching the NaCl template structure, the solid magnesium scaffold remains. The spatial resolution of the disclosed method is limited. Moreover, mechanical stability issues of the green body limit the available geometries.

Manufacturing processes used for the fabrication of inorganic materials often involve the addition to organic binders to the inorganic particles of interest and the formation of a green body that is later subjected to debinding and sintering at high temperatures. A common problem when debinding green bodies consisting of organic binder and ceramic particles is crack formation. While debinding is thought to be a common cause of cracking, polymerization shrinkage, low particle concentrations and the presence of unreacted monomer can also lead to the formation of cracks during the manufacturing process [24,25,26,27]. No general solution has been found so far to avoid such crack formation.

When such leachable structures are intended to be used as templates, the infiltration of the templates with the material of interest is an important step. There exists a variety of techniques for infiltration, such as pressure and vacuum infiltration, chemical vapor infiltration, and injection molding. Infiltration processes are also commonly used to fabricate composite materials.

For the production of metal matrix composites (MMC), mostly vacuum infiltration is used. Typically, a porous preform and the metal to be infiltrated are first evacuated in an oven before the metal melts. Then, pressurized, inert gas is filled into the oven, forcing the molten metal into the preform. The surface tension of the metal and its wettability on the preform material play an important role for the quality of the final product.

Chemical vapor infiltration works similar to the aforementioned infiltration, however the infiltrate is a reactant gas flowing through the preform (mixture of carrier gas along with the matrix material of interest). This is achieved through diffusion or a pressure difference. The matrix material reacts chemically with the preform and hence results in a composite material.

Injection molding is most popular for the processing of polymeric material but can also be used for metals and ceramics. Typically, the molten cast material is injected under high pressure into a heated mold. After cooling, the molded part is removed from the mold. The mold contains small air vents, placed at appropriate places in the mold wall, which allow the air to escape when the material is injected. Injection molding is not an infiltration technique per se but can be used as such. Vacuum pressure infiltration as well as injection molding are both techniques that can be used for the infiltration of sintered ceramic articles.

There is a general need for improvements in this field.

**Summary of the Invention**

It is the overall objective of the present invention to provide improvements in manufacturing cast objects with template molds.

One object of the present invention is to provide advantageous photopolymerizable slurries that allow to manufacture molds made of soluble inorganic salts, with higher spatial resolution and/or increased mechanical stability.

Another object of the invention is to provide advantageous methods for manufacturing such photopolymerizable slurries, methods for manufacturing sintered ceramic articles made of soluble inorganic salts, as well as methods for manufacturing cast articles.

These and other objects are substantially achieved through the features of the independent claims. Advantageous embodiments and variants are set forth in the dependent claims.

Further aspects of the present invention become evident as this description proceeds.

The present invention may comprise one or more of the features recited in the attached claims, and/or one or more of the following features and combinations thereof.

One aspect of the invention concerns a photopolymerizable slurry.

A photopolymerizable slurry according to the invention comprises a plurality of particles of an inorganic salt and at least one polymerizable monomer or oligomer. The inorganic salt has a melting point of above 250 °C at atmospheric pressure and has a solubility in water above 9 %w/w at room temperature.

The term "polymerizable monomer or oligomer" shall comprise molecules that can react together with other monomer or oligomer molecules to form a larger polymer.

The term "photopolymerizable" shall refer to the ability of a chemical composition to a polymerization reaction that requires actinic radiation (e.g. UV or visible light) for the propagation step.

As used herein, the term "polymerizable slurry" means a mixture of solid material suspended or dispersed in a liquid, of which composition at least one component can undergo polymerization upon initiation, e.g. free-radical polymerization initiation. As a result, the mixture undergoes gelation. Typically, prior to gelation, the polymerizable slurry has a viscosity profile consistent with the requirements and parameters for the additive manufacturing method it is intended to be used for (e.g. 3D printing). Irradiation with actinic radiation having sufficient energy to initiate a polymerization or cross-linking reaction, for instance ultraviolet (UV) radiation or electron beam radiation, or both, can be used for this purpose. By exposing the photopolymerizable slurry to a suitable light source, the radiation-curable monomer or oligomer can be polymerized. The resulting polymer matrix acts as a binder for the inorganic salt particles, resulting in a green body.

The term "inorganic salt" shall comprise salts consisting of inorganic ions, including carbonate and hydrogen carbonate ions.

A melting point above 250 °C of the inorganic salt allows a sufficient increase in temperature to remove the polymer binder and other carbonaceous compounds by pyrolysis, without melting the particles and destroying the binderless body.

For an efficient removal of a template mold after a casting process by dissolving the mold in water or an aqueous solution, the inorganic salt used for producing the mold should have a sufficiently high solubility, above 9 %w/w in water.

Inorganic salts that can be used for such a photopolymerizable slurries, and that have a melting point of above 250 °C at atmospheric pressure and a solubility in water above 9 %w/w at room temperature are listed Table 1.

Table 1: Salts

| Name | Formula | Melting point [°C] | Solubility in water [g/100g water] |
|---|---|---|---|
| Barium bromide | $BaBr_2$ | 857 | 100 |
| Barium chloride | $BaCl_2$ | 961 | 37 |
| Barium iodide | $BaI_2$ | 711 | 221 |
| Beryllium chloride | $BeCl_2$ | 415 | 71.5 |
| Beryllium sulfate | $BeSO_4$ | 1127 | 41.3 |
| Cadmium bromide | $CdBr_2$ | 568 | 115 |
| Cadmium chloride | $CdCl_2$ | 568 | 120 |
| Cadmium sulfate | $CdSO_4$ | 1000 | 76.7 |
| Calcium bromide | $CaBr_2$ | 742 | 156 |
| Calcium chloride | $CaCl_2$ | 775 | 81.3 |
| Calcium iodide | $CaI_2$ | 783 | 215 |
| Cesium chloride | $CsCl$ | 646 | 191 |
| Cobalt chloride | $CoCl_2$ | 737 | 56.2 |
| Copper(II) bromide | $CuBr_2$ | 498 | 126 |
| Copper(II) chloride | $CuCl_2$ | 598 | 75.7 |
| Iron(II) bromide | $FeBr_2$ | 691 | 120 |
| Iron(II) chloride | $FeCl_2$ | 677 | 65 |
| Lead(II) nitrate | $Pb(NO_3)_2$ | 470 | 59.7 |
| Lithium bromide | $LiBr$ | 550 | 181 |
| Lithium chloride | $LiCl$ | 610 | 84.5 |
| Lithium iodide | $LiI$ | 469 | 165 |
| Lithium nitrate | $LiNO_3$ | 253 | 102 |
| Magnesium bromide | $MgBr_2$ | 711 | 102 |
| Magnesium chloride | $MgCl_2$ | 714 | 56 |
| Manganese(II) bromide | $MnBr_2$ | 698 | 151 |

(continued)

| Name | Formula | Melting point [°C] | Solubility in water [g/100g water] |
|---|---|---|---|
| Manganese(II) chloride | $MnCl_2$ | 650 | 77.3 |
| Manganese(II) bromide | $MnBr_2$ | 698 | 151 |
| Manganese(II) chloride | $MnCl_2$ | 650 | 77.3 |
| Nickel(II) bromide | $NiBr_2$ | 963 | 131 |
| Nickel(II) chloride | $NiCl_2$ | 1031 | 67.5 |
| Potassium bromide | KBr | 734 | 25 |
| Potassium carbonate | $K_2CO_3$ | 899 | 111 |
| Potassium chloride | KCl | 771 | 25 |
| Potassium cyanide | KCN | 622 | 69.9 |
| Potassium fluoride | KF | 858 | 102 |
| Potassium iodide | KI | 681 | 148 |
| Potassium nitrate | $KNO_3$ | 334 | 38.3 |
| Potassium nitrite | $KNO_2$ | 438 | 312 |
| Potassium sulfate | $K_2SO_4$ | 1069 | 12 |
| Rubidium chloride | RbCl | 724 | 93.9 |
| Silver fluoride | AgF | 435 | 172 |
| Sodium bromide | NaBr | 747 | 94.6 |
| Sodium carbonate | $Na_2CO_3$ | 856 | 30.7 |
| Sodium chloride | NaCl | 802 | 36 |
| Sodium cyanide | NaCN | 562 | 58.22 |
| Sodium nitrate | $NaNO_3$ | 306.5 | 91.2 |
| Sodium sulfate | $Na_2SO_4$ | 884 | 28.1 |
| Zinc bromide | $ZnBr_2$ | 402 | 488 |
| Zinc chloride | $ZnCl_2$ | 325 | 408 |
| Zinc iodide | $ZnI_2$ | 450 | 438 |

It is also possible to use mixtures of inorganic salts. Interactions of different salt compounds, particularly interactions of particles of different salts, can influence the local and/or the overall behavior of the salt particles, particularly the melting temperatures, and the sintering behavior.

Advantageously, the used inorganic salt consists of ions that are physiologically acceptable. For example, sodium, potassium, calcium, and magnesium can be used as cations, and chloride and carbonate can be used as anions. Sodium chloride (NaCl) and potassium chloride (KCl) are particular advantageous choices for the inorganic salt in view of physiological safety, melting temperature, solubility in water without the need of organic solvents, costs, biocompatibility, high thermal and chemical stability, and applicability to a vast range of scaffold materials.

The polymerization reaction of the monomer in the photopolymerizable slurry can be initiated by irradiation with suitable actinic radiation.

Advantageously, the photopolymerizable slurry can comprise a photoinitiator.

A photoinitiator is a molecule that creates reactive species (free radicals, cations or anions) when exposed to actinic radiation (UV or visible light). The photoinitiator must be suitable for the applied system of polymerizable monomer or oligomer,

By irradiation of a certain volume of the photopolymerizable slurry with light, the polymerization reaction in this volume is initiated, and the photopolymerizable slurry in this volume solidifies.

In an advantageous variant of a photopolymerizable slurry according to the invention, the inorganic salt particles are coated or functionalized with a dispersant.

This surface coating or functionalization of the inorganic salt particles is advantageous in regard to constant and reproducible properties of the slurry and the green body produced from it.

An advantageous variant of a photopolymerizable slurry according to the invention further comprises an inhibitor.

The inhibitor can for example be a UV blocker, a compound that absorbs UV light. This is advantageous in regard to increased spatial resolution during the additive manufacturing process.

The inhibitor can be a chemical additive that inhibits or retard the degradation (oxidation, thermal degradation, etc.) of the polymerizable slurry or the solidified green body. This is advantageous e.g. in regard shelf life, mechanical stability of the polymer matrix during debindering, etc.

An advantageous variant of a photopolymerizable slurry according to the invention further comprises a diluent.

A diluent can be used to adjust the viscosity of a photopolymerizable slurry.

A diluent can also be used to improve the mechanical stability of the green body or the binderless body.

At room temperature, the diluent can be a liquid, or a solid compound that is dissolved in another component of the photopolymerizable slurry, e.g. in a liquid monomer.

Advantageously, camphor is used as the diluent.

An advantageous variant of a photopolymerizable slurry according to the invention further comprises a sintering aid.

Advantageously, the sintering aid improves the sintering step. For example, $Na_2SO_4$ can be used to decrease the melting point of NaCl used as the inorganic salt.

Another aspect of the invention concerns a method for manufacturing a photopolymerizable slurry.

A method for manufacturing a photopolymerizable slurry according to the invention comprises the steps:

a) providing a plurality of particles of an inorganic salt in the form of a powder; wherein the inorganic salt has a melting point of above 250 °C at atmospheric pressure; and wherein the inorganic salt has a solubility in water above 9 %w/w at room temperature;

b) providing at least one polymerizable monomer or oligomer; the polymerizable monomer or oligomer being in the liquid phase; and

c) adding the inorganic salt particles to the liquid composition and mixing the inorganic salt particles with the liquid composition; obtaining a photopolymerizable slurry.

In an advantageous variant of such a manufacturing method according to the invention, the inorganic salt particles are produced by milling an amount of inorganic salt together with a dispersant in a liquid phase, thereby obtaining a dispersion of inorganic salt particles coated or functionalized with the dispersant; and removing the liquid phase.

The milling may for example be carried out in a suitable ball mill.

The liquid phase can be removed for example by evaporation of the liquid phase, or by spray drying.

In an advantageous variant of such a manufacturing method according to the invention, a photo initiator, and/or an inhibitor, and/or a diluent, and/or a sintering aid are provided.

More advantageously, the provided photo initiator, inhibitor, diluent, or sintering aid are in a liquid state, or are solids that are dissolved in the monomer or oligomer, obtaining a liquid composition.

A further aspect of the invention concerns a method for manufacturing a sintered ceramic article.

A method for manufacturing a sintered ceramic article according to the invention comprises the steps:

a) providing a photopolymerizable slurry according to the invention;

b) selectively curing the photopolymerizable slurry to obtain a green body article;

c) debinding the green body article to obtain a binderless body article; and

d) sintering the binderless body article to obtain a sintered ceramic article.

The term "binderless body" designates a green body after debinding, namely the removal of the binder, i.e. the polymer matrix, e.g. by pyrolysis. The removal of the binder matrix can be incomplete. The rest of the binder will burn off during the sintering step.

The remaining polymer can help to connect the inorganic salt particles until in the subsequent sintering step the individual particles are sintered together. Thus, a binderless body may comprise less than 10%, advantageously less than 5%, even more advantageously less than 2% of the original weight of the polymer in the green body.

In an advantageous variant of such a manufacturing method according to the invention, the selective curing of the photopolymerizable slurry to obtain a green body article is carried out within an additive manufacturing process.

Advantageous additive manufacturing methods that can be applied for the additive manufacturing process in such a manufacturing method are for example stereolithography (SLA) 3D printing, 2-photon polymerization (2PP), or digital light projection (DLP) 3D printing.

Another aspect of the invention concerns a green body article as it can be obtained after step b) of a method for manufacturing a sintered ceramic article according to the invention.

Another aspect of the invention concerns a binderless body article, as it can be obtained after step c) of a method for manufacturing a sintered ceramic article according to the invention.

Another aspect of the invention concerns a sintered ceramic article, manufactured with a method for manufacturing a sintered ceramic article according to the invention.

A further aspect of the invention concerns a method for casting articles.

A method for manufacturing cast articles according to the invention comprises

a) providing a first template mold; wherein the first template mold comprises a sintered ceramic article according to the invention;

b) providing a second mold; wherein the second mold comprises a compartment into which said first template mold can be placed;

c) mounting the first template mold into the compartment of the second mold, thereby obtaining an operative mold;

d) casting a fluid casting material into said operative mold to obtain after solidification of said casting material an infiltrated template mold comprising a solid article that is at least partially located within the first template mold; and

e) separating said solid article from the first template mold by dissolving the sintered ceramic article of the first template mold with a suitable solvent, for example water.

Since the first template mold is destroyed in the separation step, the first template mold is a so called lost mold.

In an advantageous variant of such a manufacturing method according to the invention, the solid article and the first template mold are removed from the second mold before dissolving the sintered ceramic article of the first template mold.

In another advantageous variant of such a manufacturing method according to the invention, the solid article is positively locked in the first template mold.

In a further advantageous variant of such a manufacturing method according to the invention, the second mold is a permanent mold or a lost mold.

In yet another variant of such a manufacturing method according to the invention, a metallic material or a polymer material or a ceramic material or a composite material is used as the casting material.

Another aspect of the invention concerns cast solid articles, manufactured with a method according to the invention as discussed above.

Such a cast article can be a tissue scaffold and/or a medical implant and/or a medical device.

**Brief description of the drawings**

In order to facilitate a fuller understanding of the present invention, reference is now made to the appended drawings and figures. These references should not be construed as limiting the present invention and are intended to be exemplary only.

Components that are identical, or that are identical at least in terms of their function, are designated below by identical or similar reference numbers.

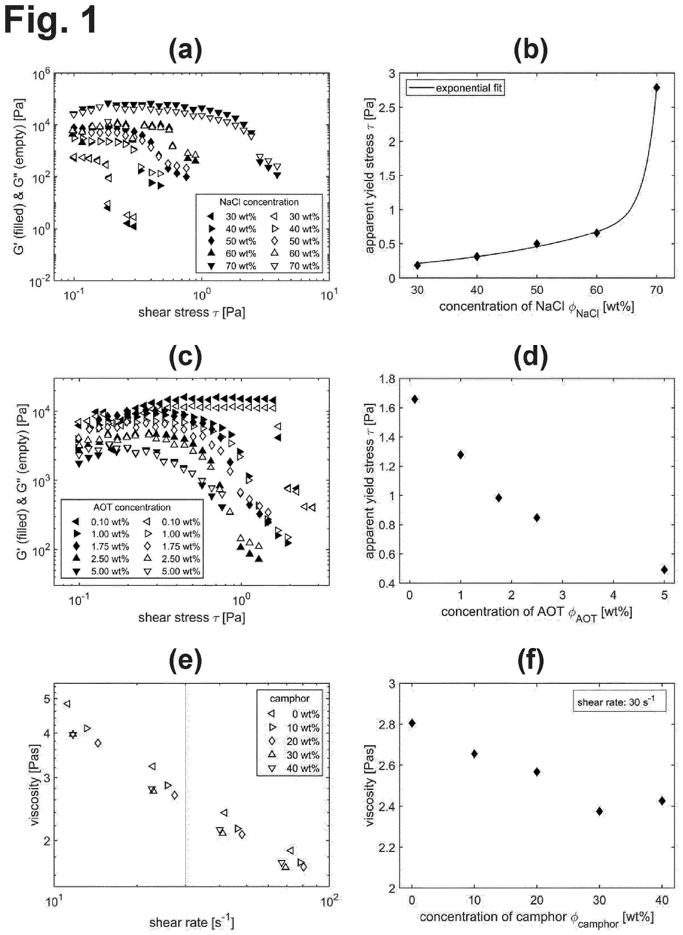

Figure 1    shows the results of rheological measurements on various printing resins: (a) Amplitude sweeps in oscillation of printing resins with an increasing NaCl concentration; (b) apparent yield stresses were extracted from (a) and plotted against the concentration of NaCl; (c) oscillatory rheological results of printing resins containing increasing amounts of a dispersant AOT; (d) apparent yield stresses extracted from (c) plotted against the dispersant concentration; (e) rheological data of the stress-controlled steady state measurement of printing resins with varying amounts of a diluent camphor; (f) viscosity values extracted from measurement (e) at a shear strain of 30 $s^{-1}$.

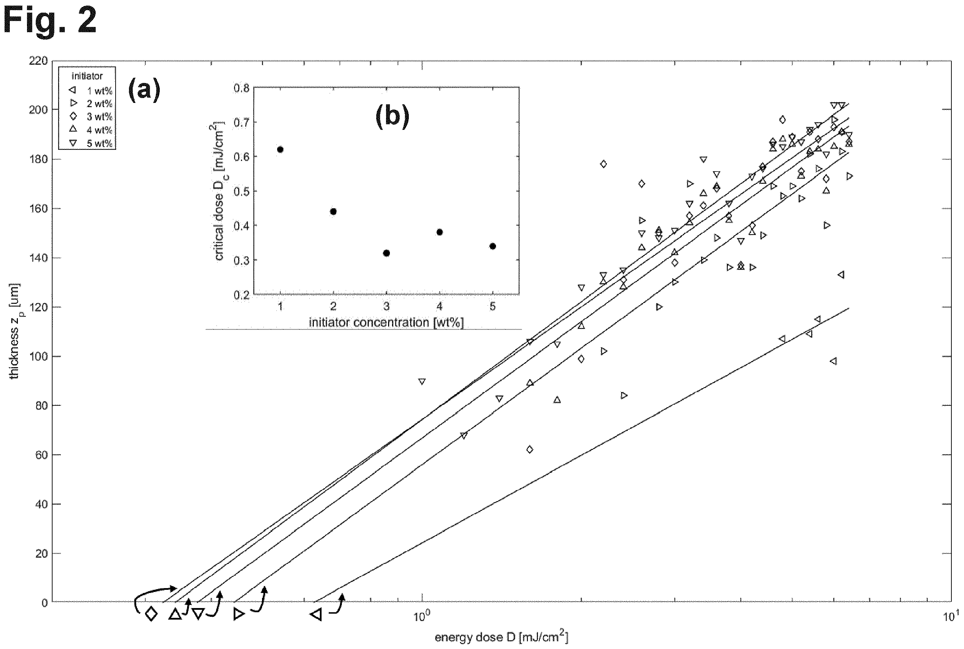

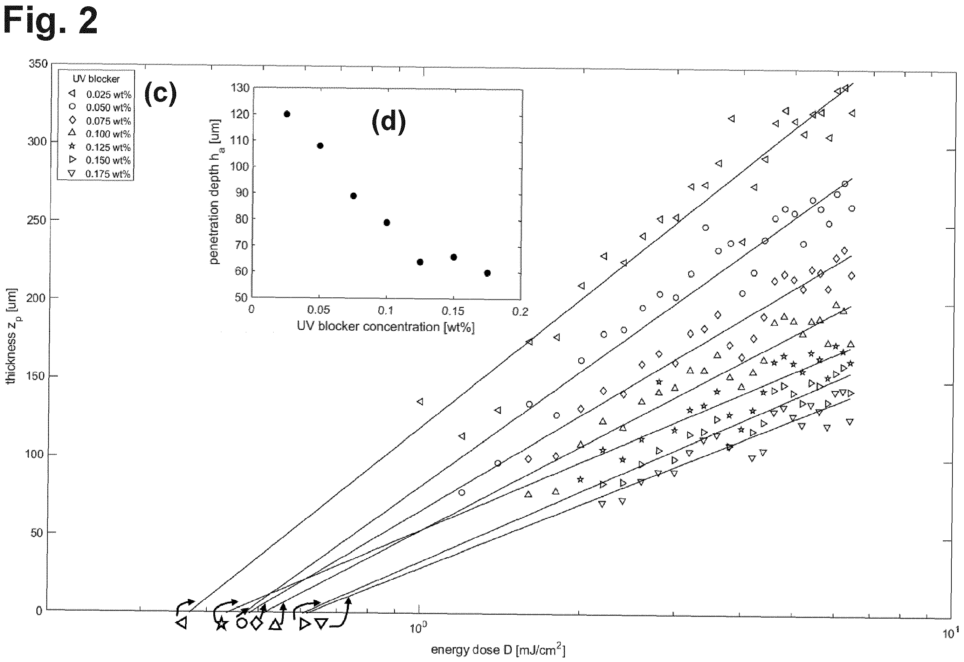

Figure 2      shows the results of exposure tests for different photoinitiator and UV blocker concentrations. (a) The layer thickness $z_p$ after exposure is plotted against the applied energy dose $D$ during the exposure, for different concentrations of photoinitiator. (b) The critical energy dose $D_c$ determined from the extrapolated fits in (a) are plotted against the photoinitiator concentration. (c) The layer thickness $z_p$ after exposure is plotted against the applied energy dose $D$ during the exposure, for different concentrations of UV blocker. (d) The penetration depth $h_a$ determined from the extrapolated fits in (c) are plotted against the UV blocker concentration.

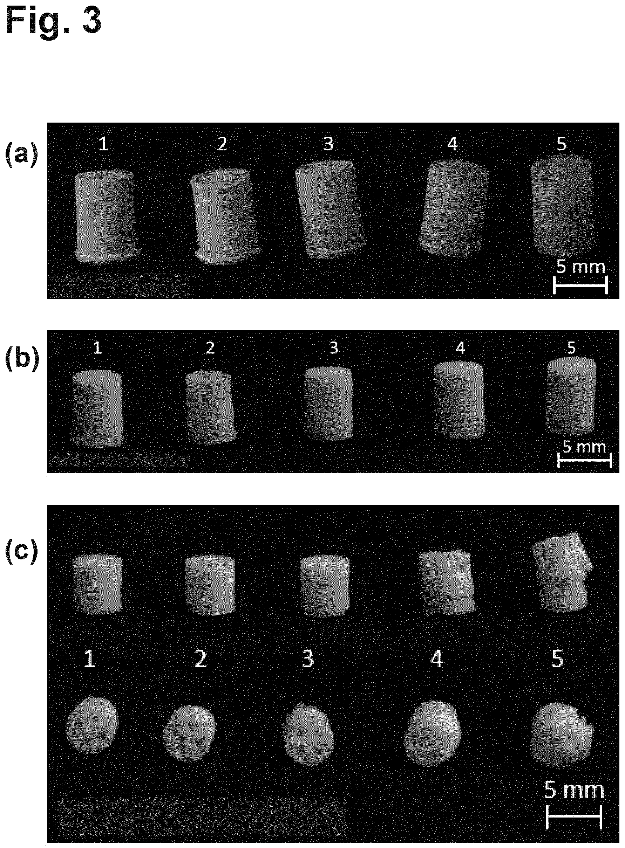

Figure 3      shows originally identical sample objects subject to different post-printing washing routines according to Table 5, the printing resin used to print these samples containing only HDODA as monomer: (a) images of green bodies, (b) images of sintered bodies. (c) depicts similar sample objects after sintering, using a printing resin containing 85 wt% IBA and 15 wt% TMPTA as monomers.

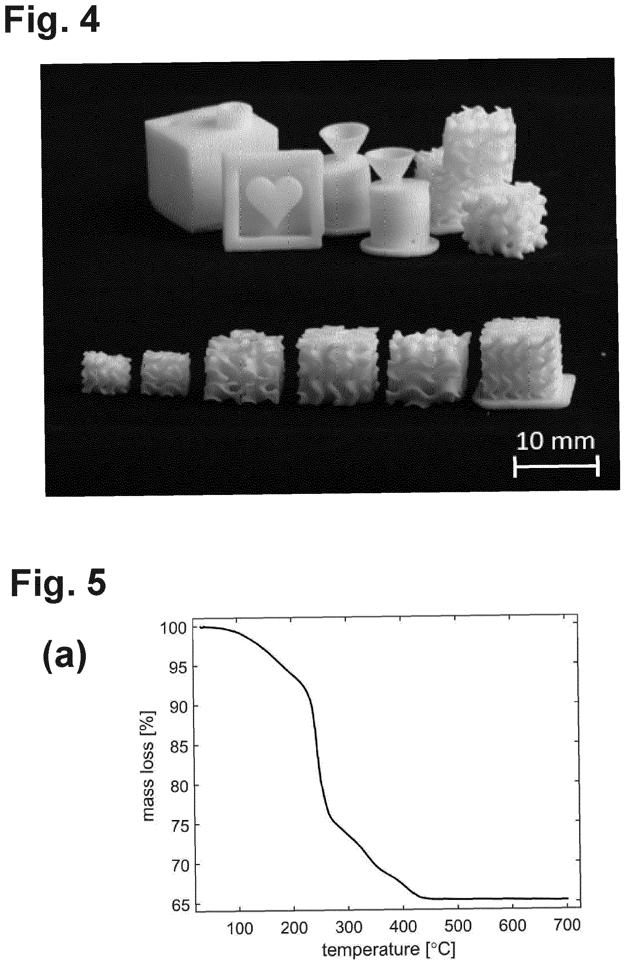

Figure 4      shows photographs of exemplary sintered NaCl molds manufactured according to the invention, including closed and open molds and gyroid shaped templates.

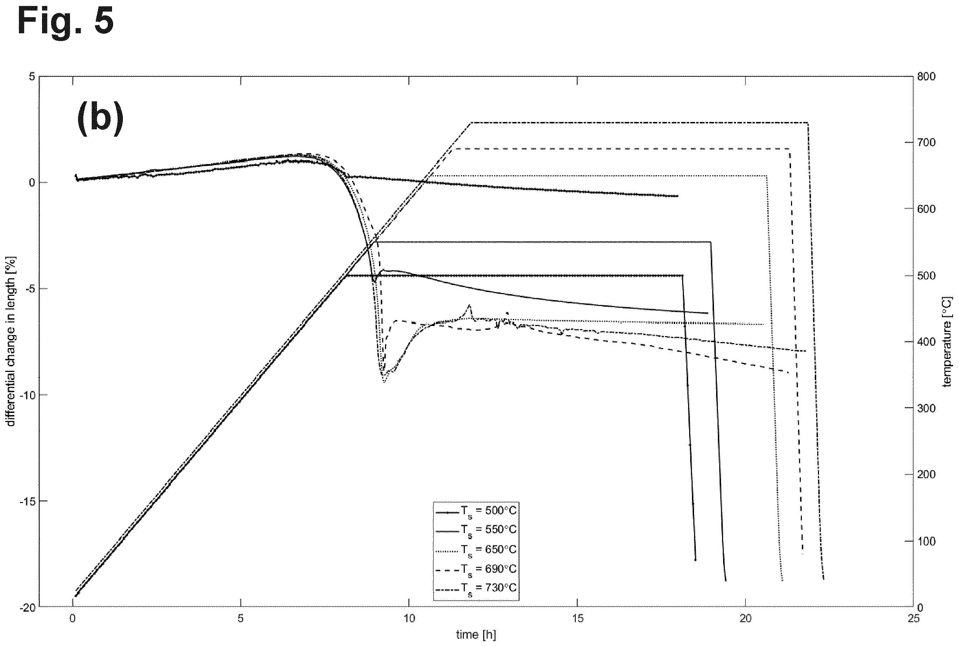

Figure 5      shows (a) the results of thermogravimetric analysis of a printed part showing the mass loss as a function of temperature, and (b) the results of dilatometry for different sintering temperature, with the differential change in length and the temperature displayed as left and right y axis, respectively, as a function of time.

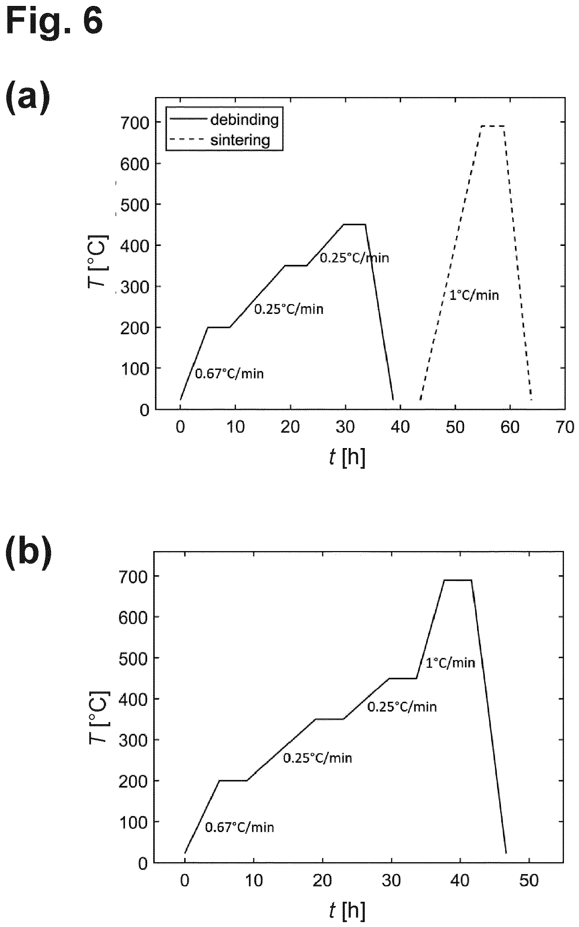

Figure 6      shows exemplary debinding and sintering cycles, (a) with debinding (solid line) and sintering (dashed line) performed in separate cycles, and (b) with a combined debinding and sintering cycle.

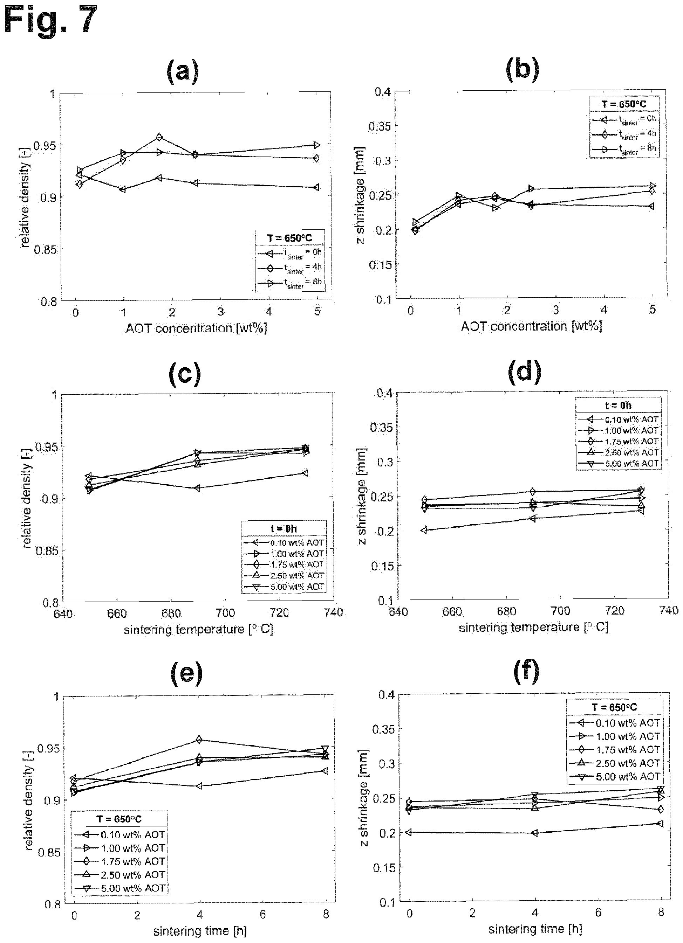

Figure 7      shows the influence of sintering parameters on relative density and shrinkage. (a) and (b) show the relative density and the shrinkage as a function of AOT concentration. (c) and (d) display the relative density and the shrinkage as a function of the sintering temperature. (e) and (f) depict the relative density and the shrinkage as a function of the sintering time.

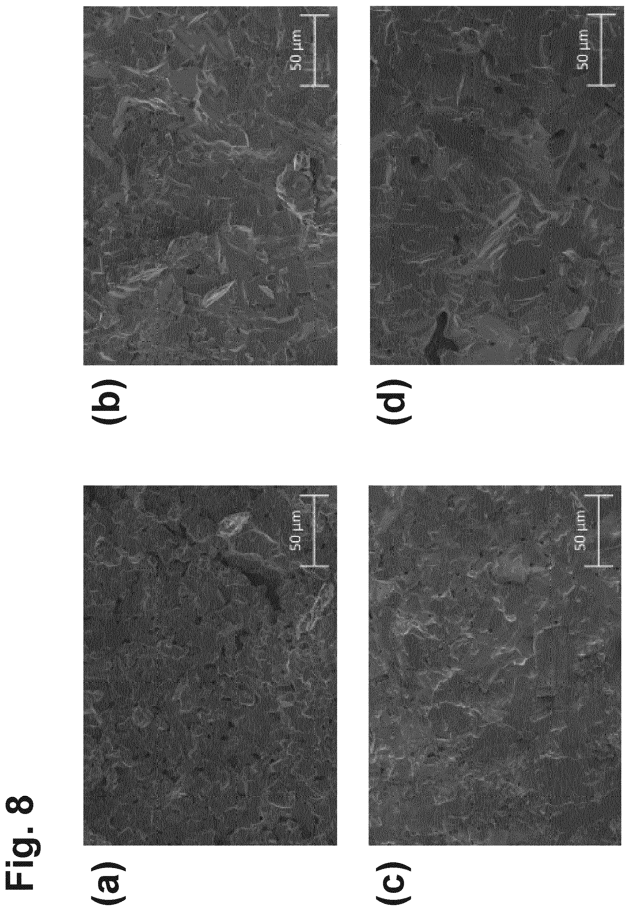

Figure 8      shows SEM images illustrating the influence of the sintering parameters on the microstructure of a sintered NaCl mold, namely the influence on the density and surface quality of the sintering temperature and sintering time for different AOT concentrations. (a) 0.1 wt% AOT, $t_s = 0$ h, $T_s = 650$ °C, (b) 0.1 wt% AOT, $t_s = 8$ h, $T_s = 650$ °C, (c) 5 wt% AOT, $t_s = 0$ h, $T_s = 650$ °C, (d) 0.1 wt% AOT, $t_s = 0$ h, $T_s = 730$ °C.

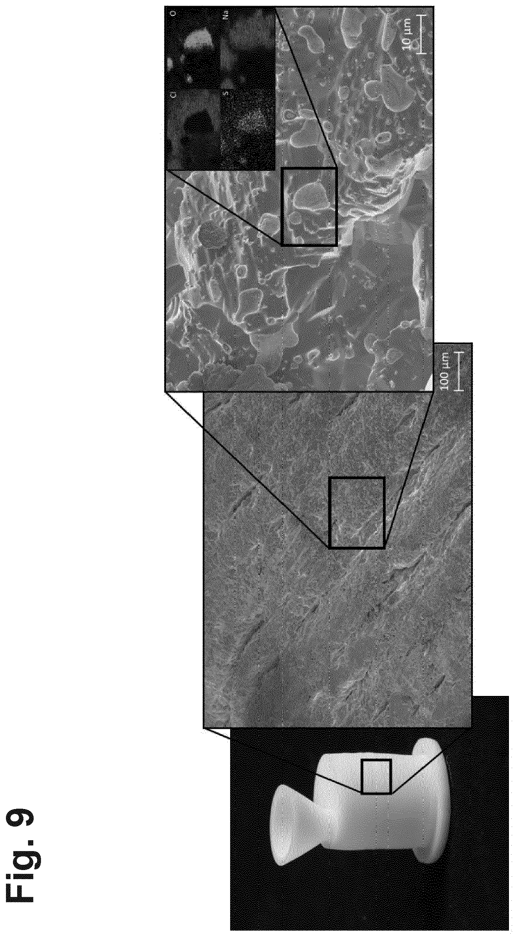

Figure 9      shows a sintered, printed NaCl structure (left) with SEM images of its microstructure with increasing magnification (two middle images) and EDS measurements (right) for Na, S, O and Cl.

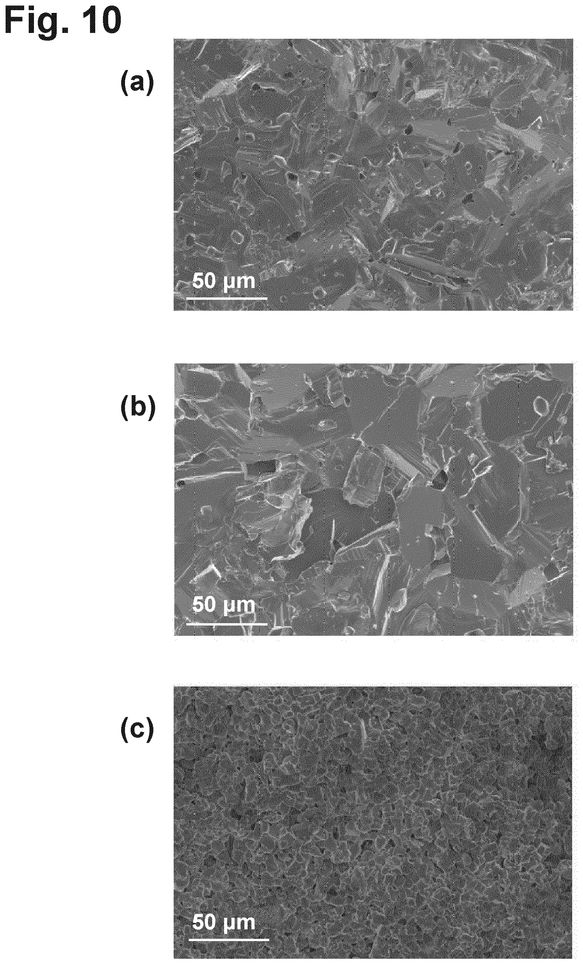

Figure 10     shows the influence of the dispersant AOT and the sintering aid $Na_2SO_4$ on the microstructure of sintered NaCl bodies. (a) 1.75 wt% AOT (with respect to NaCl), 0 wt% $Na_2SO_4$; (b) 0 wt% AOT, 1.85 wt% $Na_2SO_4$; (c) 0 wt% AOT, 0 wt% $Na_2SO_4$.

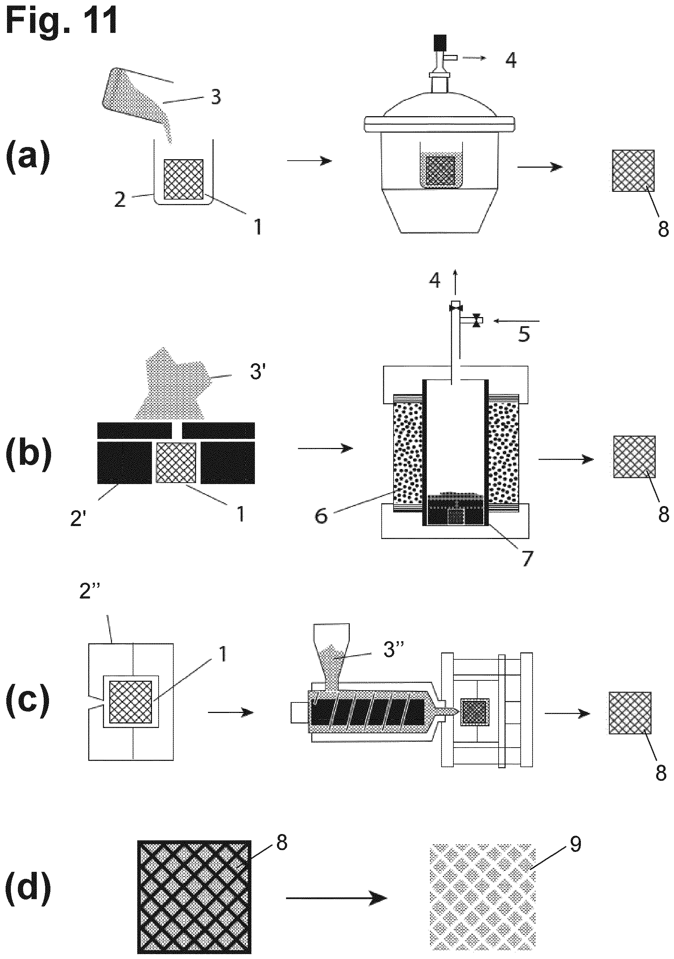

Figure 11     schematically shows exemplary methods for the infiltration process: (a) casting in a beaker and vacuum assisted infiltration in a desiccator; (b) casting at elevated temperature in a melting set-up, where the NaCl mold is placed in an outer mold and casting is performed by application of an external overpressure; and (c) injection molding; while (d) schematically shows the removal of the template mold.

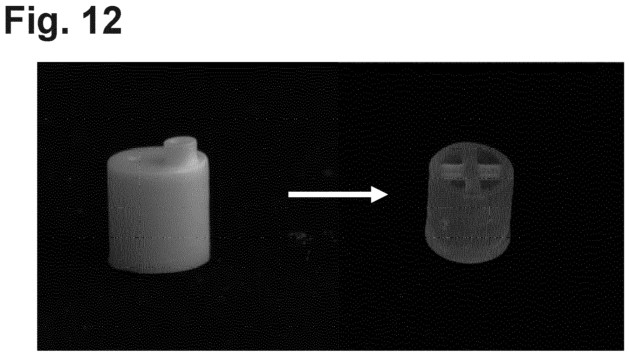

Figure 12     shows photographs of a sintered NaCl mold manufactured according to the invention (left) and a cast epoxy part made with said mold, after infiltration and dissolution of the NaCl mold (right).

**Detailed description of the invention**

A process to prepare a salt-based slurry for photopolymerization is described.

The obtained slurry is gelled by radiation curing, particularly in a DLP stereolithography printing step, and a green body is obtained. Non-gelled slurry is further removed, such as by rinsing and/or sonication in a solvent with low salt solubility. The cleaned article is further heat treated, so that the polymer matrix of the green body is pyrolyzed, and a binderless object consisting essentially of inorganic salt particles is obtained. The binderless article is then sintered to obtain a mechanically stable mold with a dense surface. During sintering potential residuals of the binder are removed. The sintered mold is used as a template for infiltration with a fluid material such as a powder, liquid or molten material, which can be further solidified by temperature, light or chemical reaction. Alternatively, the sintered article can also be used as a core for dip coating, spray coating or wrapping. Finally, the sintered mold is dissolved by an appropriate polar solvent, preferably an aqueous solution, and the final cast product is obtained.

Materials used

The following compounds have been used for the tests and experiments described in this description:

- Camphor (($\pm$)1,7,7-trimethylbicyclo[2.2.1]heptan-2-one, CAS No. 76-22-2, Alfa Aesar)
- Sodium bis(2-ethylhexyl) sulfosuccinate, also commonly called sodium dioctyl sulfosuccinate (AOT) (CAS No. 577-11-7, 98 %, Sigma Aldrich)
- Ethanol (absolute for analysis, Merck KGaA, Germany)
- Ethylene glycol (for analysis, Merck KGaA, Germany)
- Phenyl-bis-(2,4,6-trimethylbenzoyl)-phosphinoxide (BAPO) (CAS No. 162881-26-7, Irgacure 819, BASF)
- Isopropanol (99.8%, Sigma Aldrich)
- Sodium chloride, NaCl (CAS No. 7647-14-5, Sigma Aldrich)
- Olive oil
- 1-Phenylazo-2-naphtol (Sudan I, CAS No. 842-07-9, Sigma Aldrich)
- 1,1,1-Trimethylolpropane triacrylate (TMPTA) (CAS No. 15625-89-5, 98,5%, abcr GmbH)
- 1,6-Bis(acryloyloxy)hexane (HDODA) (CAS No. 13048-33-4, >85% GC, Tokyo Chemical Industry Co., Ltd.)
- Isobornyl acrylate (IBA, CAS No. 5888-33-5, 93 %, abcr GmbH)

Additive manufacturing

Additive manufacturing (AM, also known under the synonymous term 3D printing) is a manufacturing technique where an object is built up layer by layer in an additive manner. It enables the production of highly complex structures, which cannot always be achieved with conventional manufacturing techniques. Various additive manufacturing techniques have been developed over the past years, such as direct ink writing (DIW), selective laser melting (SLM), and stereolithography (SL). Stereolithography is the method that is advantageously applied for the given invention.

To produce an object with additive manufacturing, a three-dimensional model of the object, for example in the form of computer-aided design (CAD) file, is sliced into a series of 2D cross-sectional layers before it can be printed. In stereolithography, each of these thin layers of photocurable resin will be cured consecutively on top of each other. The resin usually consists of photosensitive monomers such as acrylates or epoxides, which will polymerize upon illumination [27, 28].

Stereolithography can be divided into two classes, with the main difference being how the actinic radiation (typically UV light) is applied. Either selected parts of the whole layer are cured at once using a light mask, or a laser beam scans over the surface, only polymerizing small volumes, so called strands, one by one. These two methods are called projection-based stereolithography (PSL), also known as direct light processing (DLP), and scanning-based stereolithography (SSL), respectively. SSL cannot reach a resolution as high as PSL because the scanning laser beam has an under width limit. Moreover, PSL is less time consuming because each layer is solidified in one step. However, SSL has higher radiation intensities and is advantageous for larger objects, although some of the resolution is lost.

A stereolithography apparatus (SLA), generally comprises five parts: resin tank, recoater, print platform, optics, and control systems. The recoater is mainly needed for high viscosity resins because they do not flow back and level in the tank. The printing platform is coupled to an elevator which controls the up- and downward movements of the platform while printing. The light source is usually a laser (Diode, He-Cd, Ar) coupled to an acoustic optical modulator to quickly turn on and off the laser. For direct light processing, usually a LED-LCD system is used as the light source.

In this description, a stereolithography apparatus will alternatively be named as stereolithography printer or 3D printer.

The photopolymerizable resin used for stereolithography will alternatively be named as photopolymerizable slurry, photocurable resin, printing resin, or 3D printing ink.

There are two possible setups for a stereolithography printer, namely top-down or bottom-up.

For the top-down orientation, the light source is placed on top, and the printing platform is inserted in the tank just below the resin surface. After each exposure, the platform moves downward by one layer height. The resulting objects are thus printed in upward direction. This approach has some disadvantages such as the need for large amount of resin and the extensive time for the resin to equilibrate again after the stage moved downward. Further, it is very challenging to control the layer thickness because only gravitational forces act on the fresh layer of resin on top of the already cured one. The use of the recoater blade is crucial in mitigating this problem as well as the use of a rather low-viscosity resin.

In contrast, the bottom-up approach circumvents most of these issues. The light source is placed on the bottom and the print head is placed on top. The printing platform is moved to the bottom of the resin bath where a thin layer of resin is polymerized on the platform surface through a transparent, anti-adhesive window in the bottom of the tank. After illumination, the tank is tilted in order to detach the cured layer from the tank and to allow the resin to flow back and form a fresh layer on the tank bottom. This allows for the use of considerably less resin compared to the top-down approach. After each layer, the print head is moved upwards by one layer height to cure the next layer. The resulting objects are hanging from the print head upside down. Another advantage compared to the top-down setup is the avoidance of a height limit of the object due to the tank size. The layer thickness can be controlled better, because it depends on the platform elevator and not on the fluid properties of the resin, which leads to higher vertical resolution as well as better surface quality of the object. However, the major drawback of the bottom-up approach is the requirement for an anti-adhesive and transparent bottom surface of the tank. Coatings such as Teflon or silicone are needed to easily detach the cured resin from the bottom, while it continues to adhere to the print platform.

In order to print complex geometries with high spatial resolution, it is crucial to understand and analyze the influence of the fundamental parameters in stereolithographic printing that determine the width and depth of the exposed layer and how they depend on the applied energy dose. There are two groups of parameters, a first group related to the printing process such as the laser power, layer thickness and hatch spacing, and a second group determined by the resin, including penetration depth and critical energy dose.

During printing, the photoinitiator turns into a radical upon irradiation with a suitable light source and hence initiates a free radical polymerization reaction of the monomer. This reaction continues as long as the resin is illuminated and will lead to a soft solid once the gelation point of the resin is reached. The intensity of the light decreases in $z$ direction and xy direction of the resin away from the focal point on the surface. The gelation point can only be reached if the energy dose at that depth $D(z)$ is above a critical energy dose ($D_c$). The polymerized thickness $z_p$, also called cure depth, is the depth $z$ at which the energy dose is just sufficiently high that the resin composition reaches the gelation point.

The absorption of the energy of the light beam in the resin follows a modified version of the Beer-Lambert law:

$$z_p = h_a \ln (D/D_c), \tag{1}$$

where $D$ is the applied energy dose, $z_p$ is the resulting cure depth, $h_a$ is the penetration depth (the cure depth when $D=D_c$), and $D_c$ the critical energy dose.

The parameters $D_c$ and $h_a$ depend on the resin composition and the spectrum of the applied actinic radiation and should be determined before printing, to assess the optimal printing parameters for a certain composition. Similarly, photoinitiator and UV blocker concentrations can be optimized for a given energy dose and a desired layer thickness.

The use of a UV blocker in a photocurable resin allows to reduce the penetration depth $h_a$ and xy resolution for a given critical energy dose $D_c$, since part of the actinic radiation is absorbed by the UV blocker when passing the resin. The decreased penetration depth $h_a$ leads to a decreased production speed, since either the illumination time has to be increased to reach the same polymerized thickness or more layers have to be produced. However, the decreased penetration depth also reduces the sensitivity of the cure depth towards slight changes of the light intensity. Such changes can occur for example due to clouding of the non-adhesive film of the resin tray or due to slight variations in the illumination time, changing illumination intensity and time, respectively. Therefore, an intermediate UV blocker concentration is often desired in the resin to accelerate the printing process without making the resin too sensitivity to unavoidable variations in illumination intensity.

The monomer conversion rate depends on the depth $z$ (distance from illuminated surface), as predicted from equation (1). The degree of polymerization is largest at the surface and decreases exponentially thereafter. This irregularity within each layer can cause problems in further processing steps due to shrinkage during polymerization. As a result, mechanical stress is present and deformations appear, which may lead to crack formation of the objects. This defect is known as print-through. Furthermore, there needs to be some overcure, meaning that each strand should slightly penetrate the adjacent layers in order to avoid layer delamination, i.e. the separation of adjacent printed layers. It is advantageous to overcure 10%-35% in $z$ height to avoid common printing defects such as delamination or print-through.

For a transparent resin, a cured line of the resin has the same shape as the intensity distribution of the light source, which corresponds to a Gaussian curve. However, if the resin contains inert particles, as in the photopolymerizable slurries according to this invention, the profile of the cured line is altered significantly due to scattering of the light beam. This leads to a broadening of the beam, especially for large differences in the refractive index of the particles and the monomeric phase [27, 29]. Hence, a lower resolution is obtained. This effect can be mitigated for example by matching the refractive indices of the particles and the monomer, reducing the solid loading and adjusting the size distribution of the particles.

Method: In the context of the invention, 3D printing of the structures was carried out with a commercial bottom-up direct light processing (DLP) device (Original Prusa SL1, Prusa Research a.s.). The objects were designed in Solidworks (Solidworks 2020-2021, Dassault Systemes SolidWorks Corporation) and further edited in the original slicer software of the 3D printer (PrusaSlicer Version 2.2.0, Prusa Research a.s.).

Before printing, the printing resin was always vortexed until it was well homogenized (vortex-genie 2, Scientific Industries, Inc.). Alternatively, the printing resin can be homogenized in a mixer (Thinky Mixer ARE-250, Thinky cooperation).

The 3D printer was calibrated prior to every use and the illumination time was set to 40 s for the first four layers and to 22.5 s for the consecutive layers. The layer thickness was set to 50 $\mu$m.

The required illumination time heavily depends on the slurry composition such as type, concentration and reactivity of monomer, initiator and inhibitor. The illumination time can be calculated from the working curve measured from an exposure test of the slurry according to a pre-established protocol [30].

The printing was performed at room temperature. However, slight heating may be applied to further decrease the viscosity facilitating the printing process.

Particle preparation

Particles of commercially inorganic salts, such as for example regular NaCl particles, are generally not suitable to be directly incorporated into a 3D printing ink, because the particle size exceeds the printing layer height, which is usually between 25 $\mu$m and 100 $\mu$m. Therefore, the particle size needs to be decreased.

In ceramic stereolithography (CSL), it is common to use particles with a size range of 0.05 $\mu$m to 10 $\mu$m. Such particle size ranges can be readily obtained by planetary ball milling. The advantage of ball milling over a direct synthesis of particles with the required size is the high efficiency of ball milling, and the limited possibilities for synthesizing salt particles with a certain, small particle size.

Typically, ceramic milling balls are used to produce enough kinetic energy to break the particles into smaller ones. The size of the milling balls has a large influence on the resulting particle size and particle size distribution. Finding the optimum diameter of milling balls is a compromise between the kinetic energy available for breaking the particles and the number of contact points of milling balls where particles can be broken. For a given weight of milling balls, the number of milling balls $n$ increases with decreasing ball diameter $d,$ with the relation $n \propto d^{-3}$. The number of contact points between the milling balls can be understood as the coordination number $N$ arising from studies about dense packing of spheres. Since the coordination number is independent of the sphere diameter, the number of milling balls for a fixed weight of milling balls can be assumed to be proportional $n \propto N$. This results in the combined relation $n \propto N \propto d^{-3}$. The kinetic energy depends on the mass of the milling balls as well as the rotation speed of the ball mill. It is advantageous to use a mix of various milling ball sizes, in order to get $\mu$m sized particles with a narrow particle size distribution. Nevertheless, all the milling parameters have to be considered to find the optimal ball size variation for the desired particle size and particle size distribution. For the use of such particles in a resin, a narrow size distribution is favorable for high spatial resolution and a fine and smooth surface of the ceramic in the end.

In order to obtain a stable suspension and keep the particles from sedimenting and agglomerating, a surfactant or dispersant, respectively, can be used to modify the surface of the particles and to stabilize them in the liquid components of a photopolymerizable slurry. The surfactant can either be added after a (dry) milling process, or before milling. The latter case is more advantageous, since it reduces one step in the process.

In the context of this description, the terms "surfactant" and "dispersant" will be used interchangeably.

In one advantageous approach for producing homogeneous, fine-grained NaCl particles for use in a polymerizable slurry that can be used as a printing resin for 3D printing, the NaCl particles are reduced in size and are functionalized with the surfactant dioctyl sulfosuccinate sodium salt (AOT). In a typical process, the NaCl and the AOT are dispersed in isopropanol and added to an alumina milling jar. The dispersion is wet milled with zirconia mixing balls in four different sizes for 2 hours at 200 rpm with 5 min milling and 5 min break (planetary ball mill PM100, Retsch GmbH). The diameters of the mixing balls are 9.7 mm, 7.6 mm, 4.9 mm, and 2.4 mm.

For a batch of 200g NaCl, the total mass of the added mixing balls is 575 g. The obtained slurry of AOT-functionalized NaCl particles in isopropanol is dried overnight in the oven at 60 °C. For the de-agglomeration of the dried, functionalized NaCl particles, the obtained powder is dry ball milled with half of the zirconia mixing balls used for the wet milling process (5 min, 200 rpm).

Alternatively, the NaCl particles may also be produced by precipitation or spray drying [31].

Photopolymerizable slurry

A salt particle loaded, photocurable resin used for stereolithography typically consists of salt particles, a continuous monomeric phase, a photoinitiator, a UV blocker, and a dispersant. The dispersant is used to prevent the particles from agglomerating and keeping them dispersed and stable in the resin. Additionally, a diluent can be added to decrease the viscosity for easier printing [13, 24, 32].

There are two main groups of photocurable resins, namely aqueous and non-aqueous resins. Currently, mostly non-aqueous systems are used owing to several advantages such as higher strength of the green body and a lower difference between the refractive indices $\Delta n$ of the particles and the continuous liquid phase. Typically, the liquid phase mainly consists of epoxy or acrylate monomer as suitable polymer building blocks. For good printability of a particle-loaded resin with DLP stereolithography, it is important that the solid loading (i.e. the particle concentration) is sufficiently high while the viscosity of the resin is kept low. High solid loading is advantageous due to enhanced form and structure stability. High solid loading decreases crack formation, geometrical deformation, pore formation, and general shrinkage during sintering. Further, it is required to have a printing resin with a sufficiently small yield point, because the resin needs to flow back below the printing platform, in order to cure the subsequent layer. Additionally, a diluent can help to reduce the viscosity due to high solid loading. It is possible to add as much as 40 wt% diluent, this upper limit being given by the requirement to still have enough of the monomeric phase to ensure sufficient mechanical stability of the printed part and limited shrinkage.

High solid loading of the photopolymerizable slurry to be used as printing resin is required to achieve excellent structural and dimensional stability of the printed object. The solid content should be maximized while keeping the apparent viscosity below 5 Pa·s for a shear rate of 30 s$^{-1}$ [27]. In Figure 1(a), the loss modulus $G''$ and storage modulus $G'$ increase for increasing solid loading due to increasing internal forces and particle-particle interactions. Hence, higher shear stresses are required to overcome the interaction forces to make the printing resin flow. Upon stress application above the particle interaction forces, $G'$ drops below $G''$, meaning that the material changes its behavior from solid-like to liquid-like. The evolution of the apparent yield stress as a function of increasing solid loading is depicted in Figure 1(b). To ensure a shear stress well above the flow point during printing, the shear stress by the resin's own weight was calculated. Assuming an area of 1 cm$^2$, a thickness of 1 mm, and a density of $\rho_{resin}$ = 1.75 g/ml (calculated using the main constituents NaCl, IBA, TMPTA and camphor, see below Table 4), the stress $\sigma$ needs to satisfy the following condition:

$$\sigma = \frac{F}{A} = \frac{m_{ink} \cdot g}{A} = \frac{\rho_{ink} \cdot V_{ink} \cdot g}{A} = \frac{1750 \cdot 0.1 \cdot 10^{-6} \cdot 9.81}{10^{-4}}\,\mathrm{Pa} = 17.2\,\mathrm{Pa} > \sigma_{flow} \tag{2}$$

where $F$ the force acting on the surface area $A$. $\rho_{resin}$, $m_{resin}$ and $V_{resin}$ are the density, mass and volume of the resin, respectively, $g$ is the gravitational acceleration and $\sigma_{flow}$ is the flow stress. Technically, all flow points from the formulated resins are well below 17.2 Pa as depicted in Figure 1(b). By fitting Equation (2) to the experimental data, a solid NaCl loading of 65 wt% was found to meet the threshold stress ($\sigma_{flow}$) for a printing resin.

To reduce crack formation, it is advantageous to use a monomer or a monomer mixture that exhibits a low polymerization shrinkage. The polymerization shrinkage for a variety of acrylate monomers is listed in Table 2 (cf. [34])

Table 2: Polymerization shrinkage of monomers HDODA, IBA, TMPTA

| monomer | No. of acryl groups | experimental shrinkage [%] | calculated shrinkage [%] |
|---------|---------------------|----------------------------|--------------------------|
| HDODA | 2 | 14.0 | 23.8 |
| IBA | 1 | 5.5 | 11.3 |
| TMPTA | 3 | 12.0 | 28.6 |

The photopolymerizable slurries are prepared by first dissolving diluent (camphor), UV blocker (Sudan I), and photoinitiator (BAPO) in the monomer composition through vigorous stirring, followed by the addition of the functionalized NaCl particles. The slurry is then thoroughly mixed two times, for 5 min at 800 rpm (Thinky Mixer ARE-250, Thinky cooperation). A zirconia ball (d = 7.6 mm) was added to enhance the mixing process.

An exemplary composition of a photopolymerizable slurry is given in Table 3, with a possible range of alternative concentrations, and alternative compounds.

Table 3:

| | Compound | [%wt] (possible range) | Alternative compounds |
|---|----------|------------------------|------------------------|
| **Ceramic particles** | NaCl | 65 (40-85) | KCl |
| **Monomer(s)** | IBA<br>TMPTA | 21 (15-60)<br>3.7 | HDODA, ESOA, PPTTA, IDA, 2-HEA |

(continued)

|  | Compound | [%wt] (possible range) | Alternative compounds |
|---|---|---|---|
| **Diluent** | camphor | 10 (0-40) | vegetable oils, octane, decane, dodecane |
| **Photoinitiator** | BAPO | 0.5 (0.001-2) | TPO |
| **Inhibitor (UV blocker)** | Sudan I | 0.03 (0-1) | Tartrazine, Allura Red AC |
| ESOA: epoxidized soybean oil acrylate (CAS No. 91722-14-4); IDA: Isodecyl acrylate; PPTTA: Pentaerythritol-tetraacrylate (CAS No. 51728-26-8); TPO: Diphenyl(2,4,6-trimethylbenzoyl)phosphine oxide (CAS No. 75980-60-8) | | | |

Two advantageous variants of photopolymerizable slurries are discussed below. In a first variant a) 100 wt% HDODA monomer is used, while in a second variant b) a monomer mixture of 85 wt% TMPTA and 15 wt% IBOA is used.

Printing resin with both monomer compositions were first tested without diluent, a NaCl particle concentration of 60 wt%, and a photo initiator concentration of 1 wt% with respect to the monomer content.

The composition of three advantageous polymerizable slurries is given in Table 4.

Table 4: Composition of printing resin (polymerizable slurry)

| **Variant a)** | | |
|---|---|---|
|  | compound | wt% (relative wt% monomers) |
| inorganic salt particles | NaCl | 64.50 |
| monomer composition | HDODA | 24.32 (100) |
| diluent | camphor | 10.42 |
| photoinitiator | BAPO | 0.73 |
| UV blocker | Sudan I | 0.03 |
| **Variant b)** | | |
|  | compound | wt% (relative wt% monomers) |
| inorqanic salt particles | NaCl | 64.50 |
| monomer composition | IBA TMPTA | 20.67 (85) 3.65 (15) |
| diluent | camphor | 10.42 |
| photoinitiator | BAPO | 0.73 |
| UV blocker | Sudan I | 0.03 |
| **Variant c)** | | |
|  | compound | wt% (relative wt% monomers) |
| inorqanic salt particles | NaCl | 69.77 |
| monomer composition | IBA TMPTA | 25.42 (85) 4.48 (15) |
| photoinitiator | BAPO | 0.3 |
| UV blocker | Sudan I | 0.03 |

In DLP stereolithography, the resin has to fulfill two main rheological requirements. First, it should have no or only a small yield point. Additionally, a low viscosity (below 5000 mPa·s) is advantageous [33]. While these two requirements are best met for low solid loadings, high loadings are beneficial for limiting the crack formation during pyrolysis and sintering and reduce risk of (anisotropic) shrinkage. Hence, a compromise between a high solid content and a low viscosity has to be found for maximizing the solid content in the printing resin, yet ensuring good printability.

The printing resin was characterized with rheological measurements in order to check its printability. During DLP printing, the resin is required to flow back below the print head to replenish the new layer. Rheological measurements of the resins containing varying concentrations of AOT or NaCl were carried out by stress-controlled oscillatory measurements on a rheometer (Anton Paar MCR 302, Anton Paar GmbH). A stainless-steel parallel plate with a diameter of 25 mm and a gap size of 1 mm was used. The plates were sand blasted to minimize wall slip. For very low viscous resins (low NaCl particle concentration), a gap size of 0.5 mm was chosen. All amplitude sweeps were carried out at 1 rad/s with a logarithmically increasing shear stress from 0.001 Pa to 100 Pa. The apparent yield stress $\sigma_{flow}$ was determined from the crossover point from the storage modulus G' and loss modulus G" for each resin composition. For determining the viscosity of the resins with increasing diluent concentration, steady state measurements were conducted. The shear stress was logarithmically increased from 0.01 Pa to 1000 Pa. All of the rheological measurements were performed at 25 °C at a sample size $n = 1$.

Figure 1 shows the results of the rheological measurements on various printing resins, namely (a) amplitude sweeps in oscillation of resins with an increasing NaCl concentration; (b) apparent yield stresses were extracted from (a) and plotted against the concentration of NaCl; (c) oscillatory rheological results of resins containing increasing amounts of AOT; (d) apparent yield stresses extracted from (c) plotted against the surfactant concentration; (e) rheological data of the stress-controlled steady state measurement of resins with varying amounts of camphor; and (f) viscosity values extracted from measurement (e) at a shear strain of 30 s$^{-1}$.

Influence of solid loading: Oscillatory rheological data was acquired for NaCl particle concentrations varied between 30 wt% and 70 wt%. HDODA was used as photosensitive monomer in all compositions. Figure 1(c) shows the evolution of the storage modulus (G') and the loss modulus (G") as a function of the shear stress. For all these resins, G' dominates over G" at low shear stress but drops below G" at higher shear stresses. This intersection corresponds to the apparent yield stress, also called flow point. The flow point defines the stress at which the suspension changes from solid-like to fluid-like behavior. For an easy comparison between all resins, the apparent yield stress is plotted against the NaCl concentration in Figure 1(b) and ranges between 0.2 Pa and 2.8 Pa. The higher the solid loading, the higher the apparent yield stress. Up to 60 wt%, the apparent yield stress increases only slightly. Between 60 wt% and 70 wt% NaCl, there is a significant increase from 0.7 Pa to 2.8 Pa. The data was fitted with an exponential function represented as a solid line:

$$\sigma_{flow}(\varphi) = 1.552 \cdot 10^{-16}e^{0.5285\varphi} + 0.06842 \cdot e^{0.038\varphi}, \qquad (3)$$

where $\sigma_{flow}$ is the yield stress and $\varphi$ the solid loading of NaCl.

Influence of Surfactant on Flow Behavior: Because the NaCl particles are hydrophilic and have to be dispersed in the hydrophobic monomeric phase, the surfactant AOT is added to the printing resin. The surfactant modifies the surface of the particles to make them hydrophobic and hence decrease the particle-particle attractive interactions. In order to quantify the reduction of attractive interactions, the rheological behavior of the printing resin was investigated. Oscillatory amplitude sweeps were conducted to investigate the influence of the AOT concentration on the rheological properties. Figure 1(c) shows the storage and loss modulus as a function of shear stress. All compositions undergo the transition from solid-like to liquid-like behavior at shear stresses higher than 0.1 Pa. Generally, lower AOT concentrations lead to higher apparent yield stresses. This trend is most prominent for low AOT concentrations.

Figure 1(d) summarizes the apparent yield stresses from Figure 1(c) and relates them to the surfactant. It was found that increasing AOT concentration, directly decreases the storage and loss modulus. Apart from the moduli, the apparent yield stresses clearly decrease with increasing AOT concentration as displayed in Figure 1(d). In the presence of the surfactant, less shearing is necessary to overcome the internal forces holding the material together. For typical shear stresses of about 1 Pa present during DLP stereolithography printing, a concentration of 1.75 wt% AOT was determined as optimum. Although higher concentrations of AOT result in even lower apparent yield stresses, it is not beneficial for the system, since this can lead to bubble formation and defects of the printed parts.

Influence of Diluent on Flow Behavior: The composition of the printing resin is a compromise between high solid loading and low viscosity. Since the solid content strongly increases the viscosity of the printing resin, a diluent was added to counteract this effect. Camphor was used as diluent. An advantage of camphor is its melting temperature above room temperature and its quick dissolution in acrylates. Further, the printed parts gain additional strength after the solidification of camphor after the polymerization reaction. Data from steady state rheological measurements were acquired to investigate the influence of camphor as a diluent on the viscosity.

The concentration of camphor was altered from 0% to 40 wt% with respect to the total amount of liquid phase, as displayed in Figure 1(e). The viscosity decreases with increasing shear rate; hence all compositions show shear thinning behavior. For an ideal Newtonian fluid, the viscosity is independent of the shear rate. During DLP printing, shear rates of approximately 30 s$^{-1}$ arise [33]. Figure 1(f) depicts the viscosity as a function of camphor concentration at a shear rate of 30 s$^{-1}$. The viscosity varies between 2.4 Pa·s and 2.8 Pa·s and decreases with increasing camphor concentration, until a minimum is reached at a camphor concentration of approximately 30 wt%. For higher concentrations, the viscosity remains roughly constant. A concentration of 30 wt% camphor was identified as best because it resulted in the lowest viscosity and did not hinder the polymerization during the printing process. The use of camphor allowed the use of a high solid loading while keeping the viscosity sufficiently low.

The photoinitiator and UV blocker concentrations on the polymerization reaction have to be adjusted to maximize the resolution of the 3D printed parts, while avoiding print-through and delamination. While the initiator dominates the energy needed to initiate the polymerization, the UV blocker primarily affects the penetration depth.

To find the optimal photoinitiator and UV blocker concentrations for the photopolymerizable slurry, exposure tests have been carried out. Printing resins with a composition according to Table 4 have been used. Regular glass slides were covered with a piece of fluorinated ethylene propylene (FEP) film and further placed directly onto the liquid crystal display (LCD) of the 3D printer (Original Prusa SL1, Prusa Research a.s.). A sufficiently thick layer of printing resin was evenly spread on top of the FEP film, fully covering the glass slide. A rectangular area was split into 32 small rectangles which were exposed to near UV light ($\lambda$ = 405 nm) for different time periods, between 2 s and 64 s, with 2 s increments. After exposure, excess resin was removed, and the glass slides were rinsed with isopropanol and water. They were left to dry overnight before the thickness of each rectangle was measured with a micrometer (IP 54, Helios-Preisser GmbH).

Photoinitiator Concentration: BAPO was used as a photoinitiator. The photoinitiator concentration is relevant because the photoinitiator generates the free radicals for the polymerization reaction. If the initiator concentration is too low, the printing resin will not solidify or will not form a sufficiently deep layer, increasing risk of delamination or print-through. Contrarily, if there is too much initiator, overcuring takes place and reduces the resolution of the final part.

The photoinitiator (BAPO) concentration was varied from 1 wt% to 5 wt% with respect to the monomer concentration. The layer thickness of each rectangle was determined after exposure according to the exposure method explained above. The energy dose was obtained by multiplying the exposure time with the light intensity of the light source (0.1 mW/cm$^2$). Figure 2(a) shows the polymerized layer thickness $z_p$ as a function of the applied energy dose $D$ in a semi-Log plot. The measured thickness values range from 70 $\mu$m to 200 $\mu$m. The data points were linearly fitted, and the critical energy dose $D_c$, which corresponds to the intersection with the x-axis, was extrapolated. It should be noted that the slope corresponding to the penetration depth of the fit does not vary much with increasing photoinitiator concentration in the system (see Equation (1)). As displayed in Figure 2(a), the thickness of the polymerized layer strongly increases when the BAPO concentration increases from 1 wt% to 2 wt%. Thereafter, it still increases with an increasing amount of initiator, but not significantly. This is due to more initiator molecules available resulting in a higher number of radicals, which initiate the polymerization reaction. Similarly, the thickness increases for higher radiation energy doses. An overall trend was detected and fitted with a line (Figure 2(a)). Below $D_c$, no polymerization takes place.

Figure 2(b) shows $D_c$ as a function of photoinitiator concentration. The critical energy dose $D_c$ varies significantly with changing photoinitiator concentrations. It ranges from 0.32 mJ/cm$^2$ for 3 wt% to 0.62 mJ/cm$^2$ for 1 wt%. First, $D_c$ decreases with increasing initiator concentration. This is due to more reactive molecules that can initiate a polymerization reaction. Above 3 wt%, $D_c$ levels off. Thus, increasing the photoinitiator initiator concentration above 3 wt% does not result in an earlier initiation of the polymerization reaction. Since there are no beneficial effects of higher initiator concentrations, 3 wt% was chosen as optimum for this system.

UV Blocker Concentration: Due to the light scattering particles, the light penetration is in principle limited also in absence of a UV blocker. However, the very high penetration depth in such a system directly results in high sensitivity of the cure depth to slight process changes in illumination time or illumination intensity - both affecting the irradiation dose. Reducing the penetration depth for higher process stability will inevitably result in longer print duration. It is hence essential to find a good balance between stable and efficient printing for a desired resolution. Such a balance is usually found at a penetration depth of 2 - 4x targeted layer height, with a factor 2 favoring high resolution prints. For a layer thickness of 50 $\mu$m, a penetration depth of 100 - 200 $\mu$m is required. UV blockers must hence absorb light at the wavelength of the light used for printing. In the discussed 3D printing resins, Sudan I was used as a UV blocker.

For finding the optimal value and keeping the resolution high, exposure tests were conducted for different UV blocker (Sudan I) concentrations, in analogy to the exposure tests in Figures 2(a) and 2(b). The concentration of Sudan I was varied from 0.025 wt% to 0.175 wt% with respect to the total monomer concentration. Figure 2(c) shows the polymerized layer thickness $z_p$ as a function of the applied energy dose $D$ in a semi-Log plot. Lower UV blocker concentrations lead to a thicker polymerized layer for the same energy dose $D$ because less light is absorbed by the UV blocker. These results show that the UV blocker is indeed effective. The thickness measured of the rectangles varies from about 80 $\mu$m to 350 $\mu$m for concentrations from 0.175 wt% to 0.025 wt% of Sudan I, respectively. The data points are linearly fitted for each concentration value. Looking at the two important parameters of Equation (1), namely the x-intercept $D_c$ and the slope $h_a$, the influence of the UV blocker concentration is larger on the penetration depth $z_p$ than on the critical energy dose $D_c$. Figure 2(d) depicts the penetration depth $h_a$ as a function of the UV blocker concentration. It is clearly visible that $h_a$ significantly decreases from 120 $\mu$m to 64 $\mu$m for a concentration of 0.125 wt%. For higher concentration, the penetration depth remains roughly constant. Considering the best resolution and print speed balance, a penetration depth of 100 $\mu$m was targeted for a layer height of 50 $\mu$m. An optimal Sudan I concentration of 0.05 wt% was found to decrease the penetration depth of the slurry to 108 $\mu$m.

Specific examples of photopolymerizable slurries:

Example 1: An intermediate slurry was prepared by ball milling a mixture consisting of 28.6 g HDODA, 69.6 g NaCl, and 1.8 g AOT. To prepare the slurry, 1.7 g HDODA, 0.024 g BAPO, and 0.00013 g Sudan I were added to 10 g of the intermediate slurry. The slurry was printed at a light intensity of 20 mW/cm$^2$, with an exposure time of 3 s and a layer thickness of 50 $\mu$m.

Example 2: Surface-modified NaCl particles were prepared by ball milling NaCl with 1.75 w/w AOT in isopropyl alcohol followed by drying at 60°C overnight. A slurry was prepared by mixing 24.5 wt% HDODA, 10.5 wt% camphor, 3 wt% BAPO (with respect to monomer concentration), and surface-modified NaCl particles as balance. The critical energy dose and the penetration depth were determined from an exposure test as 0.26 mJ/cm$^2$ and 151 $\mu$m, respectively. Using a 3D printer with a light intensity of 0.1 mW/cm$^2$, an illumination time of 5 s was applied for each layer. The base layer illumination time was set to 15 s.

Example 3: Surface-modified NaCl particles were prepared as in example 2. A slurry was prepared by mixing 20.7 wt% IBA, 3.7 wt% TMPTA, 10.4 wt% camphor, 2 wt% Omnicure 819 (with respect to monomer concentration), 0.05 wt% Sudan I (with respect to monomer concentration), and surface-modified NaCl particles as balance. The critical energy dose and the penetration depth were determined from an exposure test resulting in 0.51 mJ/cm$^2$ and 119 $\mu$m, respectively. Using a 3D printer with a light intensity of 0.1 mW/cm$^2$, an illumination time of 10 s per layer was applied. The initial exposure time was 30 s.

Example 4: Surface-modified NaCl particles were prepared as in example 2. A slurry was prepared by mixing 20.0 wt% IBA, 6.7 wt% AESO, 8 wt% camphor, 3.9 wt% BAPO (with respect to monomer concentration), 0.125 wt% Sudan I (with respect to monomer concentration), and surface-modified NaCl particles as balance. The critical energy dose and the penetration depth were determined from an exposure test resulting in 0.18 mJ/cm$^2$ and 51 $\mu$m, respectively. Using a 3D printer with a light intensity of 0.1 mW/cm$^2$, an illumination time of 22.5 s per layer was applied. The initial exposure time was 30 s.

Example 5: Surface-modified KCl particles were prepared by ball milling KCl with 1.75 w/w AOT in isopropyl alcohol followed by drying at 60°C overnight. A slurry was prepared by mixing 20.7 wt% IBA, 3.7 wt% TMPTA, 10.4 wt% camphor, 2 wt% Omnicure 819 (with respect to monomer concentration), 0.05 wt% Sudan I (with respect to monomer concentration), and KCl as balance. The critical energy dose and the penetration depth were determined from an exposure test resulting in 0.63 mJ/cm$^2$ and 197 $\mu$m, respectively. Using a 3D printer with a light intensity of 0.1 mW/cm$^2$, an illumination time of 9 s per layer was applied. The initial exposure time was 30 s.

Washing after Printing

After 3D printing, excess printing resin with unreacted monomer has to be removed from the printed object, by washing the object in a solvent. Several combinations of various solvents for washing or sonication have been tested (see Table 5).

The test objects were hollow cylinders with cross-beams. After printing, the printed objects were immersed in a first solvent A, sonication was applied for 6 minutes, and the printed objects were subsequently rinsed with a second solvent B. Drying was conducted at ambient temperature and atmosphere. Moreover, the samples were dried with their openings up-side down for the remaining solvent to flow out by gravity.

Table 5: Washing routines

| Washing routine No. | Solvent A | Solvent B |
|---|---|---|
| 1 | isopropanol | isopropanol |
| 2 | 1st isopropanol 2nd water | water |
| 3 | isopropanol | water |
| 4 | ethylene glycol | ethylene glycol |
| 5 | olive oil | olive oil |

The results of the different washing routine tests and techniques were qualitatively analyzed based on the images displayed in Figure 3. The samples shown in Figures 3(a),(b) were printed with a printing resin according to Table 4(a), whereas the samples in Figure 3(c) were manufactured with a printing resin according to Table 4(b) and a solid loading of 70 wt%. The numbers ranging from 1 to 5 on each image in Figure 3 correspond to the washing routine enumerated in Table 5.

It is clearly visible that the overall quality of the sintered samples is higher with the printing resin composition with IBA 85 wt%/TMPTA 15 wt%. It is assumed that this is the result of the more advantageous cross-linking effect of TMPTA in combination with IBA (which does not cross-link), compared to the cross-linking HDODA alone.

Visual inspection revealed that washing routine No. 2 is most detrimental, and leads to thinned out walls, and largely changed dimensions (see Figures 3(a),(b)). A lot of material (NaCl) was removed during washing. The walls are very thin, deformed and look porous. The thinning of the walls can also be detected for washing routine No. 2 in Figure 3(c), but to a smaller extent. While not wishing to be bound to a specific explanation, it is postulated that sonication in water is detrimental to the green body structure, possibly to unwanted dissolution of salt particles close to the matrix surface.

The sample objects treated according to washing routines No. 4 and 5 had few small cracks and were not as stable as the sample objects treated according to washing routines No. 1 and 3.

Sample objects No. 4 and 5 in Figure 3(c) exploded during sintering because there was still a lot of unreacted monomer in the cylinder, which was not completely washed out. One reason for this effect is that ethylene glycol is not miscible with IBA, and therefore cannot completely wash out the unreacted monomer. Since olive oil is well miscible with IBA (sample object No. 4, Figure 3(c)), it is assumed that the unreacted monomer could not be removed because the olive oil was too viscous for such small and complex structures.

After visual inspection of the samples, it thus was found that washing routines No. 1 and No. 3 rendered the best results. Both routines contain sonication in isopropanol. The only difference is the rinsing after the sonication, which is done with isopropanol and water for routine 1 and 3, respectively (see Table 5).

Based on these findings, the washing routine was further improved. The most advantageous washing routine was found to be washing the printed objects with isopropanol directly after printing and sonicate them as long and as many times as needed until the isopropanol remained transparent. After extensive ultrasonication, both rinsing with either isopropanol or isopropanol followed by water worked well. The latter yielded slightly better results because less salt was crystallized on the surface upon drying.

In a particularly advantageous washing routine, the objects are washed in isopropanol for 3 to 4 times to remove the samples from unreacted monomer and lose salt particles. After ultrasonication in isopropanol for 6 min and removal from the ultrasonic bath, the objects were washed again with isopropanol, followed by water, to remove remaining salt particles on the surface. A final rinsing with isopropanol prevents recrystallization of dissolved NaCl traces on the sample surface. The objects were then left upside down for drying at ambient temperature. It is also advantageous to dry complex structures in an air stream after the last rinsing.

With such a washing method, defect-free objects as shown in Figure 4 were obtained.

Crack formation

Crack formation can occur during and after various steps of the manufacturing process. Particularly during the debinding and sintering steps, the green bodies and binderless bodies are subject to thermal and mechanical stress, which can lead to crack formation. Cracks often are oriented along the printing planes, as was determined by 3D printing sample objects in different spatial orientations.

To reduce possible sources for crack formation, the above-discussed washing routine can be applied to remove unreacted monomer compounds prior to pyrolysis, in order to avoid thermally induced polymerization of remaining monomer. It has been experimentally shown that sonication of a sample object in isopropanol for 3x 6 min strongly reduced crack formation, compared to sonication in isopropanol for 6 min.

Furthermore, post-curing with UV light can be used to react remaining monomer.

Debinding and Sintering

After 3D printing and subsequent washing the object, the polymer binder matrix holding the salt particle structure together has to be pyrolyzed to obtain the binderless body. The binderless body has to be sintered, in order to connect the particles to obtain sufficient mechanical stability for the future use as a mold.

Pyrolysis and sintering were carried out in one cycle using a conventional electrical furnace (HT 08/17, Nabertherm GmbH). However, the debinding and sintering can also be conducted in separate steps. Debinding is carried out in ambient atmosphere. Sintering can be conducted in inert, reducing or ambient atmosphere. Depending on the slurry composition and the sintering temperature, atmosphere and time, the above mentioned heat treatment will result in a partially to fully dense sintered body.

The debinding step is critical because all of the organic material has to be pyrolyzed and leaves the ceramic material as gas phase, which subjects the green body to mechanical stress and may lead to crack formation.

A heating rate that is too high does not leave enough time for the volatile decomposition products to diffuse out of the material, resulting in pressure buildup, which leads to mechanical defects such as cracking or delamination.

It is also advantageous to obtain a mold that is sufficiently dense. For a proper infiltration of casting materials with low viscosity into the molds, the mold or at least its surface has to be dense or not wettable by the casting material. Otherwise, the liquid casting material will penetrate into the NaCl microstructure resulting in poor surface quality and impeding the dissolution of the NaCl in the subsequent manufacturing step.

The optimal conditions for debinding cycle are typically determined by thermogravimetric analysis (TGA, STA 449 C, Netzsch-Gerätebau GmbH).

Isothermal treatments are usually performed at temperatures at which the differential thermal analysis (DTA) shows peaks, i.e. when the decomposition processes occur. For objects with large cross sections, diffusion of decomposition products out of the structure is hindered, resulting in stresses. A way to reduce these stresses is to add a suitable non-reacting diluent. This reduces the overall concentration of photosensitive material and leaves some voids or channels prior to the decomposition of the polymeric material, since the diluent evaporates or decomposes at lower temperatures. Moreover, remaining unreacted monomer can induce mechanical stress during debinding due to shrinkage upon heat-induced polymerization of the remaining monomer.

To find suitable conditions for the debinding and sintering cycle adjusted to the given system, several characterization methods were applied. The influence of the AOT concentration, sintering time, and sintering temperature on the final density has been investigated.

The sample objects were cylinders with a diameter of 3 mm and a height of 5 mm and were prepared from composition Variant b, Table 4.

Shrinkage during sintering was assessed by measuring the weight, height and diameter of the green body and comparing this data to the same sample after sintering. Several parameters were investigated, namely the sintering time (i.e. duration of isotherm at sintering temperature; 0 h, 4 h, 8 h), the sintering temperature (650 °C, 690 °C, 730 °C) and the surfactant concentration (0.1 wt% to 5 wt% AOT during salt particle preparation).

Thermogravimetric analysis was carried out for assessing suitable conditions for debinding, namely determining temperatures of highest mass loss, in order to determine the temperature of isotherms for the debinding profile. The decomposition reaction and products were not studied in detail. As depicted in Figure 5(a), there is a large mass loss of the sample objects around 200 °C. The mass further drops to 65 % between 250 °C and 450 °C. At higher temperatures, no further mass loss is registered, indicating the completion of the decomposition reaction.

In order to find suitable sintering parameters for this system, dilatometric data was acquired, providing information about the length change of the sample over time for a distinct temperature profile. Figure 5(b) shows the differential change in length for sintering temperatures 500 °C, 550 °C, 650 °C, 690 °C, 730 °C. Pyrolysis and sintering were carried out in one cycle using a conventional resistance furnace (HT 08/17, Nabertherm GmbH).

The temperatures at the mass loss peaks were chosen as isotherms in the pyrolysis cycle in order to assure complete polymer removal. The corresponding debinding cycle is shown in Figure 6(a). The isotherms chosen for the debinding cycle are 200 °C, 350 °C, and 450 °C, with the increase rates listed in Figure 6, and, with a holding time of 4 h at each isotherm. This gives the pyrolysis reaction and the resulting decomposition products sufficient time for diffusing out of the sample, without risking pressure buildup and crack formation. Above 450 °C, the decomposition is completed because no further mass is lost, as shown in Figure 5(a).

The sintering can be carried out separately, as indicated by the dashed line in Figure 6(a), with a sintering isotherm of 690 °C and a holding time of 4 h. More advantageously, the debinding and the sintering steps are combined to one single process, as shown in Figure 6(b).

All the differential changes in length follow the same curve up to approximately 9 h, as displayed in Figure 5(b). A relative decrease in length reflects an increase of the density. At a sintering temperature of 500 °C, the NaCl template hardly densified. Increasing the sintering temperature to 550 °C resulted in a larger change in length at around 9 h. However, the largest change in length was realized with temperatures of 650 °C and higher.

The influence of the sintering temperature and sintering time for different AOT surfactant concentrations on the relative density (in relation to the maximal density of crystalline NaCl) and shrinkage was studied.

The density of the sintered samples was determined with the Archimedes principle (Used device: XS205 DualRange, Mettler-Toledo GmbH). Ethylene glycol was used as the medium to measure the buoyancy of the samples, and hence determine their density. Using the density of NaCl (2.16 g/cm$^3$), the relative density of the sintered sample was calculated.

The experimental data in Figures 7(a) and 7(b) show that for low sintering temperatures and low AOT concentrations the relative density as well as the shrinkage in z direction is largely determined by the applied sintering time. Similarly, keeping the sintering time at 0 h (i.e. no isotherm at the sintering temperature), an increase of the sintering temperature results in an increase of the density for all AOT concentrations, as shown in Figure 7(c). It should be noted that a sintering time of 0 h does not mean that no sintering takes place, but that the holding time at the sintering isotherm is 0 h. Sintering will nevertheless take place to a certain degree close to the sintering temperature, namely during the increase of the temperature toward the sintering isotherm and the subsequent decrease in temperature.

z-shrinkage only exhibits a trend for the lowest AOT concentration and remains nearly constant for higher AOT concentrations, as can be seen in Figure 7(d). The surfactant concentration seems to have the largest influence, as shown in Figures 7(c) to 7(f).

It is assumed that $Na_2SO_4$ acts as sintering aid, yielding higher final densities compared to the influence of sintering temperature $T_s$ and sintering time $t_s$. One decomposition product of AOT during pyrolysis is sodium sulfate $Na_2SO_4$, which together with NaCl forms a eutectic composition.

Figure 8 supports the same findings. Scanning electron microscopy (SEM) was used to obtain morphological information of the resulting microstructure surface of the NaCl molds. Imaging was performed at 5 kV with an InLens detector. The samples were mounted on carbon stickers and sputtered with 6-10 nm Pt to enable good sample conductivity.

Figures 8(a) (with 0.1 wt% AOT) and 7(c) (with 5 wt% AOT) show the surface prior to sintering ($t_s$=0). The increased concentration of AOT surfactant in the green body leads to a denser, less porous surface already after the debinding step. Similar effects take place when sintering is applied, as shown in Figures 7(a) (with $t_s$ = 0 h) and Figure 7(b) (with $t_s$ = 8 h) or when the sintering temperature is increased, as shown in Figures 7(a) (with $T_s$ = 650 °C) and Figure 7(d) (with $T_s$ = 730 °C).

Dense structures can be obtained by optimizing any of these three parameters. However, the increase in AOT concentration renders the densest microstructure (Figure 8(c). This supports the result from the dilatometry measurements. For a higher concentration of AOT (1.75 wt% AOT was used for dilatometry), the sintering time and temperature do not considerably influence the density. This result shows that dense NaCl molds can be manufactured at lower sintering temperatures and shorter sintering times, which is advantageous in view of energy consumption and manufacturing time. For an AOT surfactant concentration of 1.75 wt%, a dense microstructure can be achieved at 650 °C for a sintering time of 4 h.

Since it is assumed that sodium sulfate $Na_2SO_4$ is responsible for the enhanced sintering, energy dispersive X-ray spectroscopy (EDS) measurements were conducted on the particles found on the NaCl mold surface to prove its presence (5 kV, LEO 1530, Zeiss). In Figure 9, it can be clearly seen that the particle of interest contains Na, O, S, but no Cl. This supports the assumption that $Na_2SO_4$ is present in the system and that it acts as sintering aid.

Further evidence of the sintering aid like behavior of AOT due to its decomposition into $Na_2SO_4$ is provided in Figure 10. Slurries without UV-Blocker were cast and cured under UV light. The samples were heat treated and sintered at 690 °C for 4 h. Figure 10(a) depicts the microstructure of a slurry prepared with 1.75 wt% AOT (with respect to NaCl). A very similar, dense microstructure is obtained for slurries with 0 wt% AOT, but with 1.9 wt% $Na_2SO_4$ (with respect to NaCl). In contrast, samples which contain neither AOT nor $Na_2SO_4$ show high porosity (Figure 10(c)). These findings further support the hypothesis, that AOT has a dual role as particle dispersant during printing, and as sintering aid after its decomposition into $Na_2SO_4$.

Casting

The infiltration or coating of the samples depend on the infiltration material and geometries. In general, the material is infiltrated by a pressure difference, with or without simultaneous temperature aid to melt a material and/or lower its viscosity. An illustrative description of varying infiltration processes is shown in Figure 11.

Low viscosity materials such as epoxy, radiation curable monomers or low viscosity silicones were infiltrated by assistance of vacuum (Figure 11(a)). In this case, the salt mold 1 is placed in a glass beaker 2, which is further filled with a low viscosity material 3. This set-up is further transferred into a desiccator for vacuum 4 application. The vacuum is maintained until no gas bubbles appear from the infiltrated material surface. The set-up is further removed from the desiccator for curing at elevated temperature or irradiation curing.

For metal infiltration, the salt mold 1 is placed into an outer mold 2' e.g. made from graphite, which in turn is placed in a graphite tube 7 inside a heating cylinder 6. This gas-tight set-up is placed inside an oven. After vacuum 4 application, the set-up is heated, and the molten metal 3' is infiltrated by the application of inert gas 5 pressure. Molten magnesium metal was casted at 700 °C and an argon pressure of 5 bar, after vacuum application (Figure 11(b)).

For more viscous materials, injection molding can be used for infiltration. Injection molding can be performed by a small hand-operated injection molding device, or by an industrial injection molding machine. For both cases, the salt mold 1 is placed into a metallic outer mold 2". Polycaprolactone 3" (PCL, $M_w$ = 40'000) was injection molded at a pressure of 2 bar. Low molecular weight polypropylene (PP) was injection molded at 60 bar (Figure 11(c)).

All methods result in an infiltrated body 8, which is further leached in aqueous solution to obtain the final part 9.

1: NaCl mold/template; first mold; 2, 2', 2": second, outer mold; 3, 3', 3": material for infiltration or casting or molding; 4: connection for vacuum application; 5: inert gas inlet for pressure casting; 6: insulation material (e.g. alumina wool); 7: crucible (e.g. graphite); 8: infiltrated template mold; 9: final part.

For carbon fiber composites, the sintered articles are wrapped with the carbon fabric and coated with an epoxy resin. The fabric was further impregnated with the epoxy by vacuum application in a vacuum bag.

Upon solidification of the materials or epoxy, the sintered salt article was removed by immersion in a polar solvent, such as water. The demolding is further facilitated by ultrasonication, solvent jetting or mechanical force.

An example of a cast epoxy resin object is shown on the right side of Figure 12, while on the left side, a NaCl mold according to the invention shown, with which the epoxy resin object has been cast.

The present invention is not to be limited in scope by the specific embodiments described herein. Indeed, various modifications of the present invention, in addition to those described herein, will be apparent to those skilled in the art from the foregoing description and accompanying drawings. Thus, such modifications are intended to fall within the scope of the appended claims. Additionally, various references are cited throughout the specification, the disclosures of which are each incorporated herein by reference in their entirety.

**References**

[1] V. Karageorgiou, D. Kaplan, Biomaterials 2005, 26(27), 5474-5491

[2] Y. Conde, J. F. Despois, R. Goodall, A. Marmottant, L. Salvo, C. San Marchi, A. Mortensen, Adv. Eng. Mater. 2006, 8(9), 795.

[3] R. Nazarov, H.-J. Jin, D. L. Kaplan, Biomacromolecules 2004, 5, 718.

[4] Q. Hou, D. W. Grijpma, J. Feijen, Biomaterials 2003, 24, 1937.

[5] A. H. Brothers, D. C. Dunand, Adv. Mater. 2005, 17, 484.

[6] R. Goodall, A. Mortensen, Adv. Eng. Mater. 2007, 9, 951.

[7] K. Lietaert, L. Weber, J. Van Humbeeck, A. Mortensen, J. Luyten, J. Schrooten, J. Magnesium Alloys 2013, 1, 303.

[8] T. A. Schaedler, W. B. Carter, Annu. Rev. Mater. Res. 2016, 46, 187.

[9] J. E. Smay, G. M. Gratson, R. F. Shepherd, J. Cesarano, J. A. Lewis, Adv. Mater. 2002, 14, 1279.

[10] G. M. Gratson, M. Xu, J. A. Lewis, Nature 2004, 428, 386.

[11] C. Minas, D. Carnelli, E. Tervoort, A. R. Studart, Adv. Mater. 2016, 28, 9993.

[12] J. T. Muth, P. G. Dixon, L. Woish, L. J. Gibson, J. A. Lewis, Proc. Natl. Acad. Sci. USA 2017, 114, 1832.

[13] Y. Lee et al. J. of Europ. Ceramic Soc. 2019, 39(14), 4358.

[14] Y. Yang, G. Wang, H. Liang, C. Gao, S. Peng, L. Shen, C. Shuai, Internat. J. of Bioprinting 2019, 5(1), 1.

[15] A. Moreno Madrid, S. Vrech, M. Sanchez, A. Rodriguez, Mater. Science Engin. C 2019, 100, 631.

[16] M. P. Staiger, A. M. Pietak, J. Huadmai, G. Dias, Biomaterials 2006, 27, 1728.

[17] F. Witte, V. Kaese, H. Haferkamp, E. Switzer, A. Meyer-Lindenberg, C. J. Wirth, H. Windhagen, Biomaterials 2005, 26, 3557.

[18] F. J. O'Brien, Mater. Today 2011, 14, 88.

[19] V. Karageorgiou, D. Kaplan, Biomaterials 2005, 26, 5474.

[20] J. Wei, J. Jia, F. Wu, S. Wei, H. Zhou, H. Zhang, J.-W. Shin, C. Liu, Biomaterials 2010, 31, 1260.

[21] M. P. Staiger, I. Kolbeinsson, N. T. Kirkland, T. Nguyen, G. Dias, T. B. Woodfield, Mater. Lett. 2010, 64, 2572.

[22] T. L. Nguyen, M. P. Staiger, G. J. Dias, T. B. Woodfield, Adv. Eng. Mater. 2011, 13, 872.

[23] N. Kleger, M. Cihova, K. Masania, A. Sudart, J. Löffler, Adv. Mater. 2019, 1903783.

[24] E. Johansson et al., Materials, 10(2), 2017.

[25] C. Paredes et al., J. European Ceramic Soc., 41(1):892-900, 2021.

[26] K. Wang et al., Advanced Materials, 32(25), 2020.

[27] S. Zakeri et al., Additive Manufacturing 35, 101177, 2020.

[28] J. W. Halloran, Ann. Rev. of Materials Research, 46:19-40, 2016.

[29] N. Travitzky et al., Advanced Engineering Materials 16(6), 729-754, 2014.

[30] P. F. Jacobs, Rapid Prototyping & Manufacturing: Fundamentals of Stereolithography, 1992.

[31] M. Moncada et al., J. Dairy Science 98, 5946-5954, 2015.

[32] S. Y. Song et al., Materials and Design 180, 107960, 2019.

[33] Y. De Hazan et al., J. of Colloid and Interface Science 337(1), 66-74, 2009.

[34] A. Moeck et al., "Shrinkage of UV Oligomers and Monomers" https://radtech.org/proceedings/2014/papers/Formulation/Moeck - Shrinkage of UV Oligomers and Monomers.pdf (obtained on 16 July 2021).

**Claims**

1.  A photopolymerizable slurry, comprising

a plurality of particles of an inorganic salt; and

at least one polymerizable monomer or oligomer;

wherein the inorganic salt has a melting point of above 250 °C at atmospheric pressure; and wherein the inorganic salt has a solubility in water above 9 %w/w at room temperature.

2.  The photopolymerizable slurry according to claim 1, wherein the photopolymerizable slurry comprises a photoinitiator, and/or an inhibitor, and/or a diluent, and/or a sintering aid; and/or wherein the inorganic salt particles are coated or functionalized with a dispersant.

3.  A method for manufacturing a photopolymerizable slurry, the method comprising the steps:

a) providing a plurality of particles of an inorganic salt in the form of a powder; wherein the inorganic salt has a melting point of above 250 °C at atmospheric pressure; and wherein the inorganic salt has a solubility in water above 9 %w/w at room temperature;

b) providing at least one radiation curable monomer; the at least one radiation curable monomer being in the liquid phase; and

c) adding the inorganic salt particles to the liquid composition and mixing the inorganic salt particles with the liquid composition; obtaining a photopolymerizable slurry.

4.  The method according to claim 3, wherein the inorganic salt particles are produced by

milling an amount of inorganic salt together with a dispersant in a liquid phase, thereby obtaining a dispersion of inorganic salt particles coated or functionalized with the dispersant; and

removing the liquid phase.

5.  A method for manufacturing a sintered ceramic article, the method comprising the steps:

a) providing a photopolymerizable slurry according to claim 1 or 2;

b) selectively curing the photopolymerizable slurry to obtain a green body article;

c) debinding the green body article to obtain a binderless body article; and

d) sintering the binderless body article to obtain a sintered ceramic article.

6.  The method according to claim 5, wherein the selective curing of the photopolymerizable slurry to obtain a green body article is carried out within an additive manufacturing process.

7.  A green body article, obtained after step b) of a method according to claim 5 or 6.

8.  A sintered ceramic article manufactured with a method according to claim 5 or 6.

9.  A method for manufacturing cast articles, the method comprising

a) providing a first template mold (1); wherein the first template mold comprises a sintered ceramic article manufactured with a method according to claim 5 or 6;

b) providing a second mold (2, 2', 2"); wherein the second mold comprises a compartment into which said first template mold can be placed;

c) mounting the first template mold into the compartment of the second mold, thereby obtaining an operative mold (1, 2, 2', 2");

d) casting a fluid casting material (3, 3', 3") into said operative mold to obtain after solidification of said casting material an infiltrated template mold (8) comprising a solid article (9) that is at least partially located within the first template mold (1); and

e) separating said solid article from the first template mold by dissolving the sintered ceramic article of the first template mold with a suitable solvent, for example water.

10. The method according to claim 9, wherein the solid article and the first template mold are removed from the second mold before dissolving the sintered ceramic article of the first template mold.

11. The method according to claim 9 or 10, wherein the solid article is positively locked in the first template mold.

**12.** The method according to any of claims 9 to 11, wherein the second mold is a permanent mold or a lost mold.

**13.** The method according to any of claims 9 to 12, wherein a metallic material or a polymer material or a ceramic material or a composite material is used as the casting material.

**14.** A cast solid article, manufactured with a method according to any of claims 9 to 13.

**15.** The cast solid article according to claim 14, wherein the cast article is a tissue scaffold and/or a medical implant and/or a medical device.

# Fig. 1

**Fig. 2**

# Fig. 2

# Fig. 3

# Fig. 4

10 mm

# Fig. 5

(a)

Fig. 5 (b)

# Fig. 6

## (a)

## (b)

# Fig. 7

Fig. 8

Fig. 9

# Fig. 10

(a)

(b)

(c)

# Fig. 11

# Fig. 12

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | |
|---|---|---|---|
| X | PAREDES CLAUDIA ET AL: "Evaluation of direct light processing for the fabrication of bioactive ceramic scaffolds: Effect of pore/strut size on manufacturability and mechanical performance", JOURNAL OF THE EUROPEAN CERAMIC SOCIETY, ELSEVIER, AMSTERDAM, NL, vol. 41, no. 1, 6 September 2020 (2020-09-06), pages 892-900, XP086314463, ISSN: 0955-2219, DOI: 10.1016/J.JEURCERAMSOC.2020.09.002 [retrieved on 2020-09-06] * abstract * ----- | 14,15 | **CLASSIFICATION OF THE APPLICATION (IPC)** INV. C04B35/515 A61L27/04 A61L27/10 A61L27/14 A61L27/40 A61L27/48 A61L27/56 B22C9/02 B22C9/04 B22D19/14 B22D31/00 C04B35/626 C04B35/628 C04B35/638 C04B38/04 C04B35/632 |
| X,D | WO 2020/046687 A1 (3M INNOVATIVE PROPERTIES CO [US]) 5 March 2020 (2020-03-05) | 1-3,5-8, 14,15 | |
| Y | * page 45; claims 1,9,10,19,20 * ----- | 4 | |
| X | CN 110 407 604 A (UNIV TSINGHUA) 5 November 2019 (2019-11-05) * claims 7-10 * ----- | 8-15 | **TECHNICAL FIELDS SEARCHED (IPC)** C04B A61L B22D B22C |
| X | US 3 616 841 A (WALZ DUANE D) 2 November 1971 (1971-11-02) * column 2, line 61 - column 3, line 32; claim 1 * ----- | 8-15 | |
| X | US 3 946 039 A (WALZ DUANE D) 23 March 1976 (1976-03-23) * claims 1,12; examples I-VII * ----- | 8-15 | |
| X | CN 112 201 386 A (UNIV NANJING TECH) 8 January 2021 (2021-01-08) | 1-3,7, 14,15 | |
| Y | * claims 1,5 * ----- -/-- | 4 | |

The present search report has been drawn up for all claims

| Place of search | Date of completion of the search | Examiner |
|---|---|---|
| The Hague | 20 December 2021 | Raming, Tomas |

EPO FORM 1503 03.82 (P04C01)

**page 1 of 2**

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/072762 A1 (NEIL JEFFREY T [US] ET AL) 29 March 2007 (2007-03-29) <br> * example 1 * <br> ----- | 4 | |

TECHNICAL FIELDS
SEARCHED      (IPC)

The present search report has been drawn up for all claims

| Place of search | Date of completion of the search | Examiner |
|---|---|---|
| The Hague | 20 December 2021 | Raming, Tomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 18 6780

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2021

| Patent document cited in search report | | Publication date | Patent family member(s) | | Publication date |
|---|---|---|---|---|---|
| WO 2020046687 | A1 | 05-03-2020 | CN 112638606 A<br>EP 3843963 A1<br>US 2021292243 A1<br>WO 2020046687 A1 | | 09-04-2021<br>07-07-2021<br>23-09-2021<br>05-03-2020 |
| CN 110407604 | A | 05-11-2019 | NONE | | |
| US 3616841 | A | 02-11-1971 | NONE | | |
| US 3946039 | A | 23-03-1976 | NONE | | |
| CN 112201386 | A | 08-01-2021 | NONE | | |
| US 2007072762 | A1 | 29-03-2007 | CA 2550481 A1<br>DE 102006045888 A1<br>JP 2007123261 A<br>US 2007072762 A1 | | 29-03-2007<br>12-04-2007<br>17-05-2007<br>29-03-2007 |

EPO FORM P0459

For more details about this annex : see Official Journal of the European Patent Office, No. 12/82

## REFERENCES CITED IN THE DESCRIPTION

**Patent documents cited in the description**

- WO 2020046687 A1

**Non-patent literature cited in the description**

- *CHEMICAL ABSTRACTS,* 76-22-2
- *CHEMICAL ABSTRACTS,* 577-11-7
- *CHEMICAL ABSTRACTS,* 162881-26-7
- *CHEMICAL ABSTRACTS,* 7647-14-5
- *CHEMICAL ABSTRACTS,* 842-07-9
- *CHEMICAL ABSTRACTS,* 15625-89-5
- *CHEMICAL ABSTRACTS,* 13048-33-4
- *CHEMICAL ABSTRACTS,* 5888-33-5
- *CHEMICAL ABSTRACTS,* 91722-14-4
- *CHEMICAL ABSTRACTS,* 51728-26-8
- *CHEMICAL ABSTRACTS,* 75980-60-8
- **V. KARAGEORGIOU ; D. KAPLAN.** *Biomaterials,* 2005, vol. 26 (27), 5474-5491
- **Y. CONDE ; J. F. DESPOIS ; R. GOODALL ; A. MARMOTTANT ; L. SALVO ; C. SAN MARCHI ; A. MORTENSEN.** *Adv. Eng. Mater.,* 2006, vol. 8 (9), 795
- **R. NAZAROV ; H.-J. JIN ; D. L. KAPLAN.** *Biomacromolecules,* 2004, vol. 5 (7), 18
- **Q. HOU ; D. W. GRIJPMA ; J. FEIJEN.** *Biomaterials,* 2003, vol. 24, 1937
- **A. H. BROTHERS ; D. C. DUNAND.** *Adv. Mater.,* 2005, vol. 17, 484
- **R. GOODALL ; A. MORTENSEN.** *Adv. Eng. Mater.,* 2007, vol. 9, 951
- **K. LIETAERT ; L. WEBER ; J. VAN HUMBEECK ; A. MORTENSEN ; J. LUYTEN ; J. SCHROOTEN.** *J. Magnesium Alloys,* 2013, vol. 1, 303
- **T. A. SCHAEDLER ; W. B. CARTER.** *Annu. Rev. Mater. Res.,* 2016, vol. 46, 187
- **J. E. SMAY ; G. M. GRATSON ; R. F. SHEPHERD ; J. CESARANO ; J. A. LEWIS.** *Adv. Mater.,* 2002, vol. 14, 1279
- **G. M. GRATSON ; M. XU ; J. A. LEWIS.** *Nature,* 2004, vol. 428, 386
- **C. MINAS ; D. CARNELLI ; E. TERVOORT ; A. R. STUDART.** *Adv. Mater.,* 2016, vol. 28, 9993
- **J. T. MUTH ; P. G. DIXON ; L. WOISH ; L. J. GIBSON ; J. A. LEWIS.** *Proc. Natl. Acad. Sci. USA,* 2017, vol. 114, 1832
- **Y. LEE et al.** *J. of Europ. Ceramic Soc.,* 2019, vol. 39 (14), 4358
- **Y. YANG ; G. WANG ; H. LIANG ; C. GAO ; S. PENG ; L. SHEN ; C. SHUAI.** *Internat. J. of Bioprinting,* 2019, vol. 5 (1), 1
- **A. MORENO MADRID ; S. VRECH ; M. SANCHEZ ; A. RODRIGUEZ.** *Mater. Science Engin. C,* 2019, vol. 100, 631
- **M. P. STAIGER ; A. M. PIETAK ; J. HUADMAI ; G. DIAS.** *Biomaterials,* 2006, vol. 27, 1728
- **F. WITTE ; V. KAESE ; H. HAFERKAMP ; E. SWITZER ; A. MEYER-LINDENBERG ; C. J. WIRTH ; H. WINDHAGEN.** *Biomaterials,* 2005, vol. 26, 3557
- **F. J. O'BRIEN.** *Mater. Today,* 2011, vol. 14, 88
- **V. KARAGEORGIOU ; D. KAPLAN.** *Biomaterials,* 2005, vol. 26, 5474
- **J. WEI ; J. JIA ; F. WU ; S. WEI ; H. ZHOU ; H. ZHANG ; J.-W. SHIN ; C. LIU.** *Biomaterials,* 2010, vol. 31, 1260
- **M. P. STAIGER ; I. KOLBEINSSON ; N. T. KIRKLAND ; T. NGUYEN ; G. DIAS ; T. B. WOODFIELD.** *Mater. Lett.,* 2010, vol. 64, 2572
- **T. L. NGUYEN ; M. P. STAIGER ; G. J. DIAS ; T. B. WOODFIELD.** *Adv. Eng. Mater.,* 2011, vol. 13, 872
- **N. KLEGER ; M. CIHOVA ; K. MASANIA ; A. SUDART ; J. LÖFFLER.** *Adv. Mater.,* 2019, 1903783
- **E. JOHANSSON et al.** *Materials,* 2017, vol. 10 (2
- **C. PAREDES et al.** *J. European Ceramic Soc.,* 2021, vol. 41 (1), 892-900
- **K. WANG et al.** *Advanced Materials,* 2020, vol. 32 (25
- **S. ZAKERI et al.** *Additive Manufacturing,* 2020, vol. 35, 101177
- **J. W. HALLORAN.** *Ann. Rev. of Materials Research,* 2016, vol. 46, 19-40
- **N. TRAVITZKY et al.** *Advanced Engineering Materials,* 2014, vol. 16 (6), 729-754
- **P. F. JACOBS.** *Rapid Prototyping & Manufacturing: Fundamentals of Stereolithography,* 1992
- **M. MONCADA et al.** *J. Dairy Science,* 2015, vol. 98, 5946-5954

- **S. Y. SONG et al.** *Materials and Design,* 2019, vol. 180, 107960
- **Y. DE HAZAN et al.** *J. of Colloid and Interface Science,* 2009, vol. 337 (1), 66-74
- **A. MOECK et al.** *Shrinkage of UV Oligomers and Monomers,* 16 July 2021, https://radtech.org/proceedings/2014/papers/Formulation/Moeck - Shrinkage of UV Oligomers and Monomers.pdf